# EUROPEAN PATENT APPLICATION

(11) **EP 3 957 299 A1**
(43) Date of publication of application: **23.02.2022**
(21) Application number: 20191365.4
(22) Date of filing: 17.08.2020
(51) Int. Cl.: A61K 9/00, A61K 9/51, A61K 41/00, A61K 49/22

(54) **LOCALIZED DELIVERY OF DIAGNOSTIC OR THERAPEUTIC AGENTS USING FOCUSED ULTRASOUND**

(71) Applicant: ETH Zurich, 8092 Zurich (CH)
(72) Inventor: OZDAS, Mehmet, 8051 ZURICH (CH); SHAH, Aagam, 8051 ZURICH (CH); JOHNSON, Paul, 8051 ZURICH (CH); YANIK, Mehmet, 8117 Fällanden (CH)

(57) **Abstract**

In a method for enhancing localized delivery of a diagnostic or therapeutic agent using focused ultrasound (FUS), ultrasound-controllable drug carriers are administered into a blood vessel. Each drug carrier comprises an ultrasound-sensitive microbubble loaded with the diagnostic or therapeutic agent. The drug carriers are aggregated inside the vessel both along a radial direction and a longitudinal direction by application of an aggregation FUS sequence. Subsequently the diagnostic or therapeutic agent is released from the drug carriers by application of an uncaging FUS sequence. The aggregation and uncaging sequences are applied using FUS below a threshold power level such that harmful cavitation is avoided, as evidenced by the absence of broadband emissions and preferably non-integer harmonics from an emission spectrum of the drug carriers. Thereby damage to the vasculature by the FUS sequence is avoided. The method can in particular be employed to deliver drugs to the brain without opening the blood-brain barrier.

## Description

### TECHNICAL FIELD

The present invention relates to a method for enhancing localized delivery of a diagnostic or therapeutic agent to a human or animal body using focused ultrasound (FUS), to an ultrasound-controllable drug carrier for use in such a method, and to a corresponding system .

### BACKGROUND OF THE INVENTION

Central nervous system (CNS) disorders arise from dysfunctions in brain networks, involving different cortical, hippocampal, amygdaloidal, striatal, thalamic, and other subfields of the brain as well as different cell-types and molecular targets in these subfields. Despite significant advancements in the understanding of CNS pathologies in recent years, translating these findings has been partly hindered by the inability to selectively manipulate discrete regions of the brain. Indeed, the most common method of treatment for CNS disorders still remains systemic administration of small molecules. Systemic treatments can cause significant side effects at the dosages required for efficacy by acting in other brain regions or organs/tissues because the receptor-binding sites targeted by drugs are almost always shared by multiple brain areas. This makes it extremely challenging to exclusively modulate pathological networks. Current methods under development to target specific brain circuits such as transcranial magnetic stimulation (TMS) or penetrating electrodes (i.e. deep-brain stimulation; DBS) either have low spatial resolution, or are highly invasive, and lack cellular and molecular specificity.

Most clinically approved neurological and neuropsychiatric drugs are small molecules that readily cross the blood-brain barrier (BBB) on their own. However, these drugs need to be prevented from crossing the BBB except within the targeted brain area to avoid undesirable off-target effects.

A promising method for localized delivery of small molecules to a targeted brain area employs focused ultrasound (FUS) to locally release the drug from an ultrasound-sensitive drug carrier.

Sirsi and Borden summarized the prior art on localized delivery of small molecules using FUS (Sirsi, S. R. & Borden, M. A., State-of-the-art materials for ultrasound-triggered drug delivery, Advanced Drug Delivery Reviews 72, 3-14 (2014)). Many different drug carrier systems have been proposed in the prior art, as well as several different mechanisms which can be used as triggers for ultrasound-mediated drug release. However, most of these known approaches either require BBB opening or are extremely inefficient.

McDannold and colleagues disclosed methods for ultrasound mediated drug delivery to the brain by controlled opening of the BBB (McDannold, N. et al., Targeted, noninvasive blockade of cortical neuronal activity, Scientific Reports 5, (2015)). However, opening the BBB can be dangerous. In particular, it can cause significant immune response and blood cell infiltration to the brain and can leave the brain open to infection. In many neurological and neuropsychiatric disorders, lifelong drug treatment is needed, and any sort of repeated (chronic) BBB opening for drug delivery might have severe consequences: In many neurodegenerative diseases, compromising the integrity of the BBB and vasculature is associated with the onset and progression of various neurological disorders. Hence, while FUS-mediated BBB opening might be acceptable for one-time acute delivery of viral vectors or other macromolecules, it is currently unclear what risks repeated BBB opening may pose for long- term treatment of neurological and neuropsychiatric disorders.

Ferrara and colleagues disclosed acoustically active lipospheres, each consisting of a small gas bubble surrounded by a thick oil shell and enclosed by an outermost lipid layer (Shortencarier, M. J. et al., A method for radiation-force localized drug delivery using gas-filled lipospheres, IEEE Transactions on Ultrasonics, Ferroelectrics and Frequency Control 51, 822-831 (2004); see also US 7,358,226 B2 and US 2007/0071683 A1). Radiation-force pulses first force the lipospheres into contact with the far vessel wall as viewed from the ultrasound transducer, and subsequently fragmentation pulses rupture the lipospheres and spray the contents of the vehicle across the endothelial surface of the vessel. The authors tried to avoid aggregation of the lipospheres that might be caused by secondary radiation forces. The FUS sequence consisted of one single 10-million-cycle, 3.0 MHz, 50 kPa pulse (radiation force) followed by three five-cycle, 1.5 MHz, 2 MPa pulses separated by 20 µs delays (fragmentation). Experiments were carried out *in vitro* or ex *vivo* only. No delivery to the brain was reported, and the effects of the pulse sequence on the BBB are unknown.

Recent studies by Airan and colleagues (Airan, R. D. et al., Noninvasive Targeted Transcranial Neuromodulation via Focused Ultrasound Gated Drug Release from Nanoemulsions, Nano Letters 17, 652-659 (2017); Aryal, M., Zhong, Q., Vyas, D. B. & Airan, R. D., Noninvasive Ultrasonic Drug Uncaging Maps Whole-Brain Functional Networks, Neuron 100, 728-738.e7 (2018); Zhong, Q. et al., Polymeric perfluorocarbon nanoemulsions are ultrasound-activated wireless drug infusion catheters, Biomaterials 206, 73-86 (2019); see also WO 2019/036253 A1 and US 2019/0201326) suggested that FUS-sensitive perfluoropentane (PFP) nanoemulsions might be used to deliver lipophilic-only compounds to the brain without opening the BBB. These methods require extremely large amounts of encapsulated drug to be injected to observe any effects (nanoemulsions containing 1 mg/kg propofol need to be injected to show similar effects to systemic injection of only 2 mg/kg propofol). As the local propofol concentration required for inhibition is miniscule (tens of nanograms), this suggests that most of the propofol content of the nanoemulsions must be released nonspecifically to the rest of the brain, which is in fact a large quantity. Indeed, the authors observe significant propofol levels in the blood plasma (in the jugular vein) immediately following FUS treatment of frontal cortex, which likely causes non-specific effects. Such plasma levels are nearly half of the systemic propofol levels necessary to observe anesthetic effects. Interestingly, the authors' method of FUS-mediated drug delivery and inhibition also shows extremely transient effects (that last 8-14 secs), reminiscent of the transient effects observed with FUS alone (without any nanoparticles). It is also possible that FUS may be enhancing (albeit non-specifically) freely circulating propofol's subsequent local delivery to the brain or may be working synergistically, as no control with free propofol and FUS has been reported by the authors.

De Smedt and colleagues disclosed liposomes tethered to microbubbles as drug carriers for ultrasound-mediated drug delivery (Geers, B. et al., Self-assembled liposome-loaded microbubbles: The missing link for safe and efficient ultrasound triggered drug-delivery, Journal of Controlled Release 152, 249- 256 (2011)). The microbubbles had a lipid shell comprising 1,2-dipalmitoyl-*sn*-glycero-3-phosphocholine (DPPC) and 1,2-distearoyl-sn-glycero-3-phosphoethanolamine-N-[PDP(polyethylene glycol)-2000] (DSPE-PEG-SPDP) filled with perfluorobutane. Doxorubicin (DOX)-loaded liposomes comprising DPPC, DSPE-PEG-maleimide and cholesterol were tethered to the microbubbles via a thiol-maleimide link. DOX was released *in vitro* by application of high-power ultrasound during 15 s. No *in vivo* experiments were reported, and it is unclear whether and how efficient release of DOX can be safely achieved *in vivo* using the disclosed methodology, let alone without compromising BBB integrity.

Liposome-loaded microbubbles are also disclosed in Luan, Y. et al., Acoustical Properties of Individual Liposome-Loaded Microbubbles, Ultrasound in Medicine & Biology 38, 2174-2185 (2012). Again, the lipid shell of the microbubbles comprised DPPC and DSPE-PEG-SPDP. The liposomes comprised DPPC, DSPE-PEG-maleimide and cholesterol. Only *in vitro* experiments were reported, and again it is unclear whether and how efficient release can be ensured *in vivo* using the disclosed methodology.

It is desirable to deliver a diagnostic or therapeutic agent locally to a region of interest in the human or animal body, e.g., to the brain, the heart, the liver, to a tumor etc., at a high concentration. It is further desirable to avoid damage to the vasculature, in particular, to the blood-tissue barrier in the vessel through which the agent is provided, in particular, damage to its endothelium. If the region of interest is a brain region, it is desirable to be able to deliver the diagnostic or therapeutic agent without opening the blood-brain barrier.

### SUMMARY OF THE INVENTION

In a first aspect, the present invention provides a method for enhancing localized delivery of a diagnostic or therapeutic agent to the human or animal body, in particular, the body of a mammal, using focused ultrasound (FUS).

The method comprises:
a) administering ultrasound-controllable drug carriers into a blood vessel of the human or animal body, each drug carrier comprising an ultrasound-sensitive microbubble, the microbubble comprising a microbubble shell and a gas encapsulated by the microbubble shell, the microbubble being loaded with the diagnostic or therapeutic agent; and
b) aggregating the drug carriers inside the blood vessel by exposing the drug carriers to radiation forces, the radiation forces being generated by applying an aggregation FUS sequence to the drug carriers in a target region.

While it has been proposed in the prior art to manipulate some types of drug carriers by radiation forces, e.g., to push them towards a vessel wall using primary radiation forces, the inventors have discovered that drug-loaded microbubbles can be readily aggregated by trapping them in the focal region of the FUS radiation *in vivo.* In particular, aggregation inside a blood vessel can readily be achieved both along a radial direction and a longitudinal direction of the vessel. This can be achieved already at surprisingly low FUS power levels (in the following sometimes also referred to as "FUS energy" for simplicity) or, equivalently, at low acoustic pressures. While the applicant does not wish to be bound by theory, it is believed that aggregation is achieved by the action of secondary radiation forces. Using this method, diagnostic or therapeutic agents (in the following also simply referred to as "drugs") can be concentrated and delivered to a desired body region with millimeter-precision.

The aggregation FUS sequence can readily cause an increase of concentration of the drug carriers by at least a factor of 2, more preferably at least a factor of 10, even more preferably at least a factor of 25, yet more preferably at least a factor of 100. An increase of concentration by more than three orders of magnitude has been observed experimentally.

In advantageous embodiments, the aggregation FUS sequence is applied at FUS power levels that are below a threshold power level at which inertial cavitation starts to occur in the target region. Inertial cavitation can be harmful to the blood vasculature. In particular, if occurring in the brain, inertial cavitation can damage the blood-brain barrier to an extent that bleeding can be caused. If inertial cavitation is absent, it is therefore more likely that the blood vasculature in the target region stays intact during application of the FUS sequence. When the FUS sequence is applied to a brain region, it is more likely that the BBB will not be damaged by the FUS sequence.

Inertial cavitation is considered to be absent if broadband emissions are essentially absent from the ultrasound emission spectrum from the drug carriers in the target region. Whether or not broadband emissions are "essentially absent" can be determined as follows: Multiple ultrasound emission spectra (power vs. frequency) from the target region are determined in the absence and in the presence of drug carriers, using identical instrumentation and signal processing. Instrumentation can include a passive cavitation detector, i.e., a hydrophone designed to capture cavitation-induced acoustic signals, an amplifier for amplifying signals from the hydrophone, and optionally a filter. Signal processing can include amplification and filtering of the signals from the hydrophone. The spectral area under each spectrum (i.e., its integral) is calculated. The range of integration preferably extends from the excitation frequency (i.e., the center frequency of the applied FUS sequence) up to 4 or 5 times the excitation frequency, excluding frequency ranges where integer- and half-integer harmonics are expected, e.g., ranges of ±10% of the excitation frequency around all integer and half-integer multiples of the excitation frequency. For instance, the range of integration preferably extends from 1.1 to 1.4, 1.6 to 1.9, 2.1 to 2.4, 2.6 to 2.9, 3.1 to 3.4, and 3.6 to 3.9 times the excitation frequency. For calculating the spectral area, power is taken on a linear scale, i.e., not on a logarithmic dB scale. Broadband emissions are considered to be present if they are statistically significant when comparing the spectral area in the presence of drug carriers to the spectral area in the absence of drug carriers. In particular, broadband emissions may be considered to be present if the p-value is lower than 0.001 when comparing the spectral area in the presence of drug carriers to the spectral area in the absence of drug carriers. Larger p-values may be applied, e.g., 0.01 or 0.05.

While inertial cavitation is to be avoided, the drug carriers may well exhibit stable cavitation under the action of the aggregation FUS sequence. However, at high FUS power levels, also stable cavitation can be harmful to the vasculature, and in the brain it can lead to opening of the BBB, albeit with less destructive potential than inertial cavitation. Harmful stable cavitation can manifest itself by the presence of non-integer harmonics in the frequency spectrum, i.e., by the presence of peaks at non-integer multiples of the excitation frequency. In preferred embodiments, non-integer harmonics are therefore also avoided, in addition to broadband emissions. In particular, in some embodiments, the aggregation FUS sequence is applied at FUS power levels below a threshold power level at which non-integer harmonics at 1.5 and 2.5 times the excitation frequency start to appear in the ultrasound emission spectrum from the drug carriers in the target region. If these harmonics are essentially absent, it can be assumed that stable cavitation is in a regime that avoids any damage to the vasculature and avoids opening the BBB.

Whether or not non-integer harmonics at 1.5 and 2.5 times the excitation frequency are "essentially absent" can be determined from multiple ultrasound emission spectra from the target region in the absence and presence of drug carriers as follows: The spectral area under each spectrum (i.e., its integral) is calculated in the ranges where said non-integer harmonics are expected, e.g., the ranges of ±10% of the excitation frequency around 1.5 and 2.5 times the excitation frequency, i.e., from 1.4 to 1.6 times the excitation frequency and from 2.4 to 2.6 times the excitation frequency. Again, for calculating the area, power is taken on a linear scale, i.e., not on a logarithmic dB scale. Non-integer harmonics at 1.5 and 2.5 times the excitation frequency are considered to be present if they are statistically significant when comparing the spectral area in the presence of drug carriers to the spectral area in the absence of drug carriers. In particular, broadband emissions may be considered to be present if the p-value is lower than 0.001 when comparing the spectral area in the presence of drug carriers to the spectral area in the absence of drug carriers. Larger p-values may be applied, e.g., 0.01 or 0.05.

For the above-mentioned statistical tests, a one-tailed, unpaired t-test with Welch's correction may be applied, even though other tests may be used.

In some embodiments, the aggregation FUS sequence comprises at least one pulse, preferably exactly one pulse. The pulse preferably causes a peak negative pressure of no more than 1 MPa in the target region. A preferred range is 0.01 MPa to 0.25 MPa in the target region.

Peak negative pressure is closely related to the electric power that is supplied to the FUS transducer. It is normally determined by calibration measurements that have been carried out by the manufacturer of the transduce, and the dependence of peak negative pressure on the electric power applied to the transducer is therefore normally known. The optimum value to choose for peak negative pressure depends on various parameters, including the exact composition and size of the drug carriers, the focal volume, the properties of the blood vessel etc. The optimum value of electric power applied to the transducer and thus the optimum value of peak negative pressure can be determined by varying the electric power supplied to the transducer while observing the emission spectrum from microbubbles in the target region. Transducer power should preferably be set lower than the threshold power level at which broadband emissions start to appear to avoid tissue damage, and even more preferably transducer power should be set lower than the threshold power level at which non-integer harmonics start to appear.

In some embodiments, the aggregation FUS sequence is applied for an aggregation period of at least 10 ms, preferably at least 100 ms, more preferably at least 200 ms. The aggregation period may range up to 3 s or more. In some embodiments, the center frequency of the aggregation FUS sequence (i.e., the "excitation frequency" of the FUS sequence) ranges from 0.1 to 3 MHz, preferably 0.25 to 2.5 MHz.

The method may further comprise releasing the diagnostic or therapeutic agent from the drug carriers after aggregation by application of an uncaging FUS sequence to the target region. Thereby, the agent is released into the target region, i.e., to the desired region of the body, after its concentration has been increased by the aggregation FUS sequence.

Preferably both the aggregation FUS sequence and the uncaging FUS sequence are applied at FUS power levels below a threshold power level at which inertial cavitation starts to occur in the target region, as manifested by the absence of substantial broadband emissions from the ultrasound emission spectrum, as detailed above. Preferably also harmful stable cavitation, as evidenced by non-integer harmonics, is avoided, as also detailed above. Thereby damage to the vasculature is not only avoided for the aggregation FUS sequence, but also for the uncaging FUS sequence. Advantageously, the applied FUS power levels are sufficiently low that non-integer harmonics at 1.5 and 2.5 times the excitation frequency are essentially absent from the ultrasound emission spectrum both for the aggregation FUS sequence and for the uncaging FUS sequence. However, the present invention is not limited to such low FUS power levels, and the uncaging FUS sequence can also be applied at higher power levels.

If the target region is a brain region, the method can comprise an additional step of applying one or more BBB-opening pulses to the target region before application of the aggregation and uncaging FUS sequences. The BBB-opening pulses may act to deliberately open the blood-brain barrier so as to allow diagnostic or therapeutic agents that cannot cross the intact BBB by themselves to cross the BBB after their release from the drug carriers.

The application of an uncaging FUS sequence that avoids inertial cavitation and preferably also harmful stable cavitation is also advantageous without a prior aggregation step. Accordingly, in a second aspect, the present invention provides a method for enhancing localized delivery of a diagnostic or therapeutic agent to the human or animal body using focused ultrasound (FUS), comprising:
a) administering ultrasound-controllable drug carriers into a blood vessel of the human or animal body, each drug carrier comprising an ultrasound-sensitive microbubble, the microbubble comprising a microbubble shell and a gas encapsulated by the microbubble shell, the microbubble being loaded with the diagnostic or therapeutic agent; and
b) releasing the diagnostic or therapeutic agent from the drug carriers by application of an uncaging FUS sequence to a target region, wherein the uncaging FUS sequence is applied at FUS power levels below a threshold power level such that broadband emissions and preferably also non-integer harmonics are essentially absent from an emission spectrum of the drug carriers in the target region.

The presently proposed method is particularly valuable for enhancing localized delivery of small molecule drugs to a selected brain region. A small molecule drug is a diagnostic or therapeutic agent that has a molecular mass of not more than 900 daltons. Many small molecule drugs are able to cross the intact blood-brain barrier without BBB opening. The presently proposed method enables highly efficient localized delivery of small molecule drugs to a selected brain region without opening the BBB. In other embodiments, the presently proposed method is employed for enhancing localized delivery of other entities that are able to cross the intact blood-brain barrier without BBB opening. For instance, the presently proposed method may be employed to enhance localized delivery of viruses or antibodies that are capable of crossing the blood-brain barrier by an active mechanism.

In some embodiments, the uncaging FUS sequence comprises a plurality of pulses and is applied for an uncaging period (tu) ranging of at least 1 ms, preferably in the range of 50 to 200 ms. In some embodiments, each pulse consists of a number of cycles (NOC) ranging from 2 to 10'000, preferably 200 to 2'000. In some embodiments, the pulses are repeated within tu at a pulse repetition frequency (PRF) ranging from 0.5 Hz to 5 kHz, preferably 10 to 500 Hz. In some embodiments, the center frequency of the uncaging FUS sequence ranges from 0.2 to 3 MHz, preferably 0.25 to 2.5 MHz. The pulses preferably have a peak negative pressure of no more than 1 MPa in the target region. A preferred range is 0.05 MPa to 0.5 MPa in the target region.

The aggregation/uncaging sequence can be repeated one or more times after a delay period. The delay period between subsequent repetitions is preferably at least 200 ms.

The method may comprise:
recording ultrasound emissions from the drug carriers in the target region;
obtaining a frequency spectrum of the recorded ultrasound emissions; and
setting FUS power levels such that broadband emissions and preferably non-integer harmonics from the drug carriers in the target region are essentially absent from the thus-obtained frequency spectrum.

In this manner it can be ensured that inertial cavitation and harmful stable cavitation are avoided.

In the following, some considerations about the drug carriers are discussed.

The microbubble shell may be composed of lipids, proteins and/or polymers. Examples of suitable shell designs are discussed in S. Sirsi and M. Borden, Microbubble Compositions, Properties and Biomedical Applications, Bubble Sci Eng Technol. 1 (2019), pp. 3-17. doi:10.1179/175889709X446507. In some embodiments, the microbubble shell is a lipid shell.

The microbubble can be loaded with the diagnostic or therapeutic agent ("drug") in a variety of different manners. For instance, the drug can be directly incorporated into the microbubble shell; it can be directly attached to an inside or outside surface of the microbubble shell, e.g., by electrostatic interactions; or it can be chemically bonded to a constituent of the microbubble shell by a covalent bond. In other embodiments, the drug can be incorporated into or attached to a microparticle or nanoparticle acting as an intermediate carrier that is in turn attached to the microbubble shell on its outside, e.g., via electrostatic forces, via a direct covalent bond to a constituent of the microbubble shell, or via a linkage such as a sulfhydryl linkage, a carbodiimide linkage or an avidin-biotin linkage. The microparticle or nanoparticle may in particular be a liposome or a micelle. Examples are provided in: H. Mulvana et al., Characterization of Contrast Agent Microbubbles for Ultrasound Imaging and Therapy Research, IEEE Transactions on Ultrasonics, Ferroelectrics, and Frequency Control, vol. 64, no. 1, pp. 232-251, Jan. 2017, doi: 10.1109/TUFFC.2016.2613991; Ibsen et al, Microbubble-mediated ultrasound therapy:a review of its potential in cancer treatment, Drug Design, Development and Therapy 7 (2013), pp. 375 - 388, DOI:10.2147/DDDT.S31564; Lentacker et al., Drug loaded microbubble design for ultrasound triggered delivery, Soft Matter, 2009, 5, pp. 2161-2170; Rychak et al., Nucleic acid delivery with microbubbles and ultrasound, Adv. Drug Deliv. Rev. 72 (2014), pp. 82-93; Sirsi et al., Lung Surfactant Microbubbles Increase Lipophilic Drug Payload for Ultrasound-Targeted Delivery, Theranostics 3(6) (2013), pp. 409-419, doi:10.7150/thno.5616; Müller et al., Augmentation of AAV-mediated cardiac gene transfer after systemic administration in adult rats, Gene Ther 15 (2008), pp. 1558-1565, https://doi.org/10.1038/gt.2008.111.

The diagnostic or therapeutic agent ("drug") can be any compound that can be used for diagnostic purposes or for therapeutic purposes or for both. The agent can be, for instance, a small molecule, a nucleic acid, an oligosaccharide, a polysaccharide, an oligopeptide, a polypeptide, a protein, a virus, an antibody, or a combination of these.

In some embodiments, each drug carrier comprises a plurality of liposomes or normal or reverse micelles tethered to an ultrasound-sensitive microbubble. In the case of liposomes, the liposomes may comprise a liposome lipid shell and the diagnostic or therapeutic agent encapsulated by the liposome lipid shell. In such embodiments, the microbubble shell advantageously also is a lipid shell. In the microbubbles, the lipid shell preferably comprises a lipid monolayer, while in the liposomes, the lipid shell is a bilayer. The lipid shell of both the microbubbles and the liposomes can essentially consist of lipids having a hydrophilic head and at least one, preferably two hydrophobic fatty acid residues, as it is well known in the art. In particular, the lipid shell can essentially consist of glycerophospholipids having two fatty acid residues. Preferably each fatty acid comprises 14 to 24 carbon atoms.

In some embodiments, the microbubbles have a mean diameter ranging from 1 to 5 µm, preferably 1 to 3 µm. If the drug carriers comprise liposomes, the liposomes preferably have a mean diameter ranging from 50 to 500 nm, preferably 80 to 250 nm.

In some embodiments, the lipid shell of the microbubbles and/or liposomes comprises lipids that are modified by a polymer, in particular, a hydrophilic polymer, e.g., polyethylene glycol (PEG). The polymer moiety is attached to the hydrophilic head of the lipid. If the lipid is a glycerophospholipid, the polymer is preferably attached to the head via the phosphate group of the glycerophospholipid. The polymer chain is preferably unbranched.

All or a portion of the polymer-modified lipid may be functionalized with a linker moiety for tethering the liposomes to the microbubbles, for instance, a maleimide moiety, a thiol moiety or another linker moiety known in the art, the linker moiety of the liposomes being complementary to the linker moiety of the microbubbles. In each case, the linker moiety may be attached to a free end of the polymer moiety. A portion of the polymer-modified lipid may be non-functionalized to reduce surface adhesion and interaction with biologics. This can help increase systemic circulation time and decrease renal clearance time.

The lipid shell of the microbubbles and liposomes may comprise a sufficient amount of lipid that is not polymer-modified (e.g., non-PEGylated lipid) to make the drug carriers highly stable during circulation in the blood, a sufficient amount of non-functionalized polymer-modified lipid (e.g., non-functionalized PEGylated lipid) to make the drug carriers circulate longer by reducing surface adhesion and interaction with biologics, and a sufficient amount of functionalized polymer-modified lipid (e.g., functionalized PEGylated lipid) to tether a sufficient amount of liposomes to induce physiological response.

In particular, the lipid shell of the microbubbles and/or liposomes may comprise:
60 to 99.8 mol%, preferably 60 to 94 mol%, of lipid that is not polymer-modified;
0.1 to 40 mol%, preferably 2 to 20 mol%, of non-functionalized polymer-modified lipid, in particular, PEGylated lipid; and
0.1 to 40 mol%, preferably 2 to 20 mol%, of functionalized polymer-modified lipid, in particular, PEGylated lipid, wherein the functionalized PEGylated lipid is functionalized with a linker moiety for linking the liposomes to the microbubbles.

Preferably the polymer moiety (e.g. PEG moiety) of the functionalized polymer-modified lipid has equal or larger molecular weight than the polymer moiety (e.g. PEG moiety) of the non-functionalized polymer-modified lipid. If the polymer moieties of the functionalized polymer-modified lipid and the non-functionalized polymer-modified lipid comprise the same repeat units (i.e., if these polymers are based on the same monomers), it is preferred that the functionalized polymer-modified lipid has an equal or larger number of repeat units (which, in the case of an unbranched polymer, is equivalent to equal or larger chain length) than the non-functionalized polymer-modified lipid. This is believed to increase the probability of a liposome actually becoming tethered to a microbubble.

In some embodiments, the lipid is modified by a PEG moiety, and the PEG moiety of the non-functionalized and functionalized PEGylated lipids has a molecular mass in the range of 1'000 to 10'000 u. For instance, the PEG moiety of the functionalized PEGylated lipid may have a molecular weight of approximately 5'000 u ("PEG5000"), while the PEG moiety of the non-functionalized PEGylated lipid may have a molecular weight of approximately 2'000 u ("PEG2000").

In some embodiments, the non-PEGylated lipid is 1,2-distearoyl-sn-glycero-3-phosphocholine (DSPC), the non-functionalized PEGylated lipid is 1,2-distearoyl-sn-glycero-3-phosphoethanolamine-polyethylene glycol (DSPE-PEG), e.g., DSPE-PEG2000, and the functionalized PEGylated lipid is DSPE-PEG that has been functionalized at the free end of the PEG moiety with a linker moiety for linking the liposomes to the microbubbles, e.g., DSPE-PEG5000-maleimide or DSPE-PEG5000-thiol.

The gas that is encapsulated by the microbubble lipid shell advantageously comprises or consists of a perfluorocarbon gas, more preferably perfluorobutane gas.

A corresponding system for enhancing localized delivery of a diagnostic or therapeutic agent to a human or animal body comprises:
a source of ultrasound-controllable drug carriers as described above, each drug carrier comprising an ultrasound-sensitive microbubble, the microbubble comprising a microbubble shell and a gas encapsulated by the microbubble shell, the microbubble being loaded with a diagnostic or therapeutic agent in one of the manners described above;
a delivery device for delivering the ultrasound-controllable drug carriers to a target region of the human or animal body
a focused ultrasound source for creating FUS radiation in the target region; and
a controller configured to operate the focused ultrasound source to apply one or more FUS sequences to the target region as described above, in particular, an aggregation and/or uncaging FUS sequence as described above.

The source of ultrasound-controllable drug carriers can be, for instance, a suspension of the drug carriers in an aqueous solvent or any other suitable formulation of the drug carriers. The source of ultrasound-controllable drug carriers can be provided in any kind of packaging, e.g., in a vial, in a syringe etc.

The delivery device can comprise, in the simplest case, a syringe in the case of one-time delivery. Advantageously, however, the delivery device is configured to permit continuous delivery over a period that may range from less than 1 s to more than 1 h. The delivery device can, for instance, comprise an infusion pump, as it is well known in the art. The infusion pump may be implantable in the human or animal body. The delivery device may further comprise a cannula for administering the drug carriers into a blood vessel in the human or animal body.

All considerations discussed above in conjunction with the method of the present invention also apply to the system of the present invention, in particular, concerning the nature, size and composition of the drug carriers, as well as all considerations relating to the aggregation and uncaging FUS sequences, in particular, concerning number of pulses, timing, frequencies, and peak pressures.

The present invention further relates to the use of the above-discussed ultrasound-controllable drug carrier for enhancing localized delivery of a diagnostic or therapeutic agent to a human or animal body, localized delivery of the diagnostic or therapeutic agent being carried out by the above-discussed method.

The present invention further relates to the above-discussed ultrasound-controllable drug carriers for use in a method for enhancing localized delivery of a diagnostic or therapeutic agent to a human or animal body as discussed above.

The present invention also encompasses the use of the above-discussed ultrasound-controllable drug carriers for the manufacture of a medicament for enhancing localized delivery of a diagnostic or therapeutic agent to a human or animal body by the method discussed above.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic sequence of diagrams illustrating the concept of focal aggregation and uncaging of ultrasound-controlled drug carriers;
- Fig. 2: shows a drug-loaded ultrasound-controlled drug carrier;
- Fig. 3: shows an enlarged view of portion III in Fig. 2;
- Fig. 4: shows a schematic diagram illustrating the AU-FUS sequence;
- Fig. 5: shows a sequence of images of UC-carriers flowing through microdialysis tubing with no FUS (left), during the aggregation sequence (middle), and during the uncaging sequence (right);
- Fig. 6: shows a diagram illustrating the deposition of small molecules *in vitro* vs. peak negative pressure, using two different AU-FUS sequences "AU₁" and "AU₂" as well as two standard FUS sequences "standard₁" and "standard₂";
- Fig. 7: shows a diagram illustrating the deposition of small molecules in vivo, using a complete *in-vivo* optimized AU-FUS sequence (left), only the aggregation sequence (middle), and only the uncaging sequence (right) of the *in-vivo* optimized AU-FUS sequence;
- Fig. 8: shows a schematic illustration of the experimental setup for *in vivo* drug delivery;
- Fig. 9: shows representative peri-stimulus time histograms (PSTHs, top, bin size 2 ms) and wEPs (bottom) upon focal aggregation of drug carriers (muscimol-loaded UC-carriers) and uncaging of muscimol from one experiment;
- Fig. 10: shows the time course of normalized wEPs (negative peak) in vM1;
- Fig. 11: shows wEP responses (negative peak), plotted as baseline vss post-treatment;
- Fig. 12: shows a diagram illustrating the time course of the normalized eLFP (negative peak) responses (VEP) in neighboring V1 (without significant connectivity with vS1), as compared to responses (wEP) in vM1;
- Fig. 13: shows a diagram illustrating the results of LC-HR-MS/MS quantification of muscimol loaded in one dose of AU-FUS treatment;
- Fig. 14: shows a diagram illustrating the time course of wEP (negative peak) in vM1 during systemic muscimol ("sys. muse.") injection (i.v.);
- Fig. 15: shows a diagram illustrating the normalized radiant efficiency of Evans blue extravasation within ROIs after AU-FUS (left) and standard-FUS (right);
- Fig. 16: shows a diagram illustrating the normalized signal enhancement by MRI-contrast agent extravasation within ROIs after AU-FUS and standard-FUS;
- Fig. 17: shows a diagram illustrating normalized mean intensity values from ROI analysis of brightfield images of IgG stained brain sections after AU-FUS (left) and standard FUS (right);
- Fig. 18: shows Fast Fourier Transform (FFT) analysis of representative responses to the standard₄-FUS sequence (part A) and FFT analysis of representative response to the standard₃-FUS sequence (part B), the insets showing details of the FFT signals at ultra-harmonics and integer harmonics;
- Fig. 19: shows FFT analysis of representative response to the AU-FUS Uncaging sequence preceded by the AU-FUS Aggregation sequence (part A) and FFT analysis of representative response to only the AU-FUS Aggregation sequence (part B), the insets showing details of the FFT signals at integer harmonics;
- Fig. 20: shows the calculated power of ultra-harmonics above baseline for the AU-FUS sequence and for the standard_{3,4}-FUS sequences; and
- Fig. 21: shows the calculated power of broadband emissions above baseline for the AU-FUS sequence and for the standard_{3,4}-FUS sequences.

### DESCRIPTION OF PREFERRED EMBODIMENTS

### Definitions

In the present disclosure, references in the singular may also include the plural. Specifically, the word "a" or "an" may refer to one, or one or more, unless the context indicates otherwise.

The expression "in particular" is to be understood as being non-limiting, referring to an example.

The term "mammal" includes both humans and non-humans and includes but is not limited to humans, non-human primates, canines, felines, rodents, bovines, equines and porcines.

The expression "administering into a vessel" is to be understood as encompassing both direct administration into the vessel of interest and indirect administration, i.e., administration into another vessel that is in fluid communication with the vessel of interest.

"Focused ultrasound (FUS)" is ultrasound radiation that is focused onto a small region in space, which is called the "target region". Transducers for generating focused ultrasound are well known in the art. Focused ultrasound radiation causes acoustic pressure oscillations in the target region. The amplitude of the pressure oscillations can be characterized by a parameter called "peak negative pressure", i.e., the maximum amplitude of the negative pressure in the ultrasound field relative to ambient pressure. The dependence of peak negative pressure in a medium on the electric power or voltage amplitude supplied to the transducer can be readily determined by calibration measurements that are well known in the art.

In the present disclosure, the term "cavitation" is to be understood as broadly relating to the expansion and contraction or collapse behavior of gas-filled bubbles in an ultrasonic field. There are two different types of cavitation: inertial cavitation and stable (non-inertial) cavitation. Inertial cavitation is when a bubble rapidly expands and violently collapses in a liquid medium. Inertial cavitation generally occurs at relatively high acoustic pressures. During an inertial collapse, the speed of the gas-liquid interface may become supersonic in the gas and/or in the liquid. These supersonic motions may produce shock waves and jets in the surrounding fluid, which will propagate outward and can be damaging to surrounding biological tissue, like the blood-tissue barrier of a blood vessel. Stable cavitation is when a bubble expands and contracts in an oscillatory manner without collapsing. Stable cavitation generally occurs at lower acoustic pressures. Inertial cavitation causes both non-integer harmonics and broadband emissions in the ultrasound emission spectrum from the bubbles. Stable cavitation does not cause broadband emissions, but may cause non-integer harmonics above a certain level of acoustic pressure.

In the present disclosure, the term "radiation force" is to be understood as relating to a force that acts onto an ultrasound-sensitive (i.e., acoustically active) object like a microbubble as a result of ultrasound radiation. "Primary radiation forces" are forces that result from pressure gradients in the incident ultrasound field. They typically act in the direction of the incident ultrasound waves, i.e., away from the ultrasound transducer that generates the incident ultrasound field. "Secondary radiation forces" are forces that result from pressure gradients in the ultrasound field that is scattered by ultrasound-sensitive objects. They can cause attraction or repulsion between the ultrasound-sensitive objects.

In the present disclosure, the term "aggregation" refers to a process in which the concentration of ultrasound-controllable drug carriers in a target region is increased. In some embodiments, this increase may lead to the formation of aggregates, i.e., "clusters" of drug carriers, in which adjacent drug carriers are so close that they essentially touch one another. In other embodiments, "aggregation" encompasses an increase of concentration without the formation of aggregates.

In the present disclosure, the term "uncaging" refers to a process in which an agent is released from a carrier that encapsulates the agent. In particular, the term "uncaging" refers to a process in which an agent is released from a liposome. Before being uncaged, the agent can be located in the interior of the liposome that is delimited by the lipid shell (e.g., if the agent is hydrophilic), or it can be located within the lipid bilayer that forms the lipid shell (e.g., if the agent is hydrophobic). In both cases, the agent is considered to be "encapsulated" by the liposome.

A "diagnostic or therapeutic agent" is a compound that can be used for diagnostic purposes or for therapeutic purposes or for both. In the present disclosure, a diagnostic or therapeutic agent is also called a "drug" for short. The agent can be, for instance, a small molecule, a nucleic acid, an oligosaccharide, a polysaccharide, an oligopeptide, a polypeptide, a protein, a virus, or a combination of these.. The term "small molecule" refers to organic compounds having a molecular weight of no more than 900 daltons.

### Concept of focal aggregation and uncaging of ultrasound-controlled drug carriers

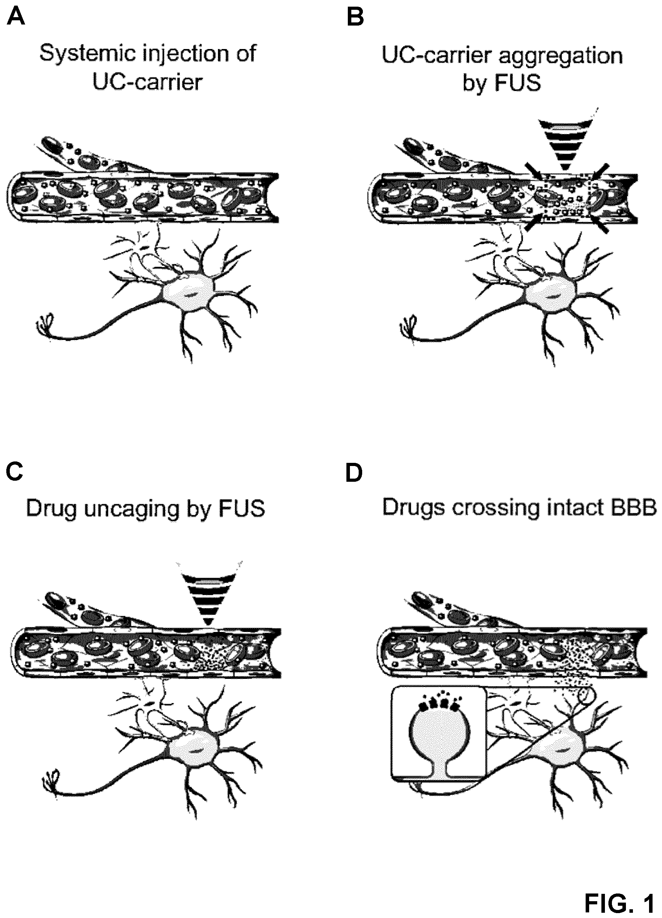

Figure 1 illustrates, in an exemplary manner, the concept of focal aggregation and uncaging of ultrasound-controlled drug carriers. (A) UC-carriers are continuously infused intravenously. (B) UC-carriers are first aggregated locally by acoustic radiation forces generated by ultrasound waves. (C) The locally concentrated UC-carriers are then uncaged to release drugs using acoustic fragmentation forces produced by ultrasound waves. (D) Small molecules diffuse across the intact BBB reaching their cognate receptors. The ultrasound beam is not to scale.

### Ultrasound-Controlled drug carriers (UC-carriers)

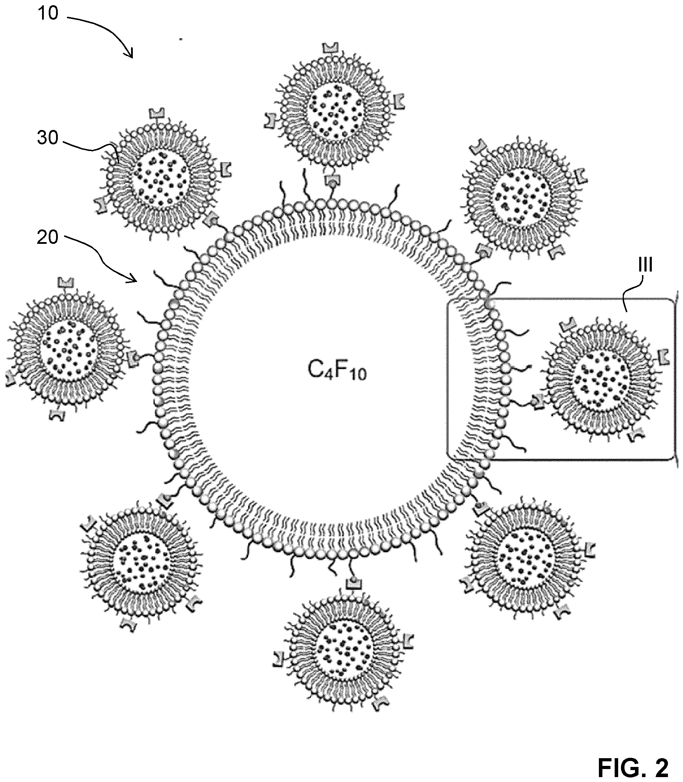

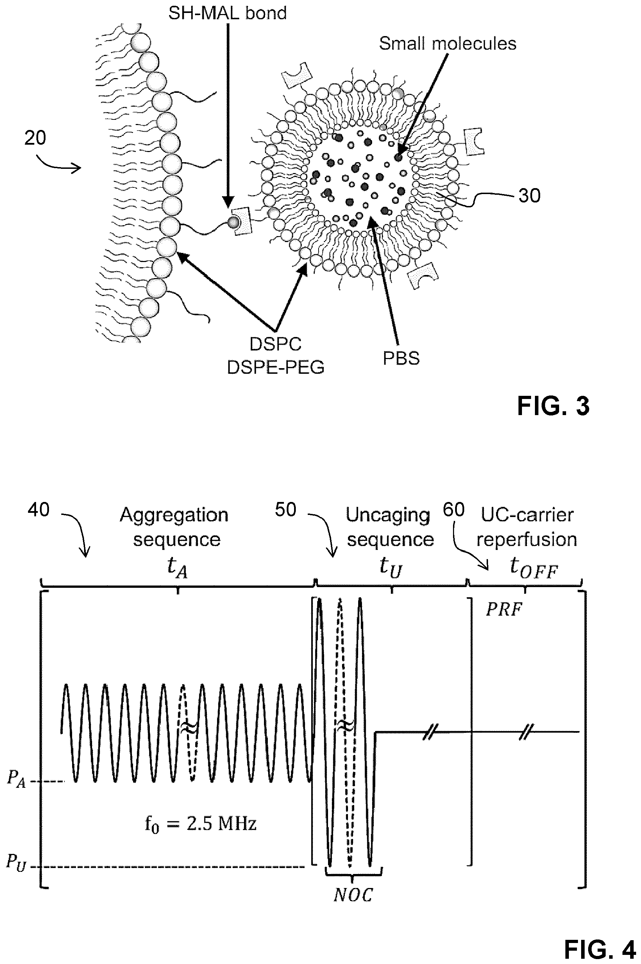

Figures 2 and 3 illustrate an exemplary ultrasound-controlled drug carrier 10. Drug-encapsulating liposomes 30 are tethered to an ultrasound-sensitive microbubble 20. Tethering liposomes to microbubbles makes liposomes responsive to ultrasound, allowing spatial control of drug deposition. The use of liposomes also allows encapsulation of diverse small molecules using either the hydrophilic liquid core or the lipophilic hydrocarbon shell.

In an embodiment, DSPC and DSPE-PEG2k formed the lipid shells of the microbubbles (monolayer) and liposomes (bilayer). The microbubbles (-1.5 µm mean diameter) were stabilized with a perfluorobutane (PFB, C₄F₁₀) gas core and had DSPE-PEG5k-Mal on the surface for conjugation with liposomes (-116 nm mean diameter; conjugated UC-carriers have -1.7 µm mean diameter), which contained DSPE-PEG5k-SH on the surface, with a PBS core. Small molecules were actively loaded into the liposomes prior to conjugation. For the experiments that will be subsequently described, either sodium fluorescein dye (model drug for *in vitro* experiments) or muscimol (for *in vivo* experiments) were loaded into the core of the liposomes.

While this embodiment has proven advantageous, the presently proposed method is expected to yield good results also for microbubbles and liposomes of different composition and with different sizes. In particular, it is expected that the presently proposed method yields good results for conjugated UC-carriers having a size ranging from about 1 to 5 µm, with liposomes having a size in the range from about 50 to 200 nm. Other lipid systems than DSPC/DSPE may be employed, and the PEG moieties do not need to be PEG2k and PEG5k, respectively. Instead of being encapsulated in a liposome, the drug may be loaded to a microbubble also in another manner.

### AU-FUS sequence

Figure 4 illustrates a combined aggregation and uncaging FUS sequence (AU-FUS sequence). The AU-FUS sequence is optimized for drug delivery with very low ultrasound pressures to avoid BBB opening. First, the aggregation sequence is applied, consisting of a single pulse with peak negative pressure ***P**_{A}* and duration ***t_{A}.*** This is immediately followed by the uncaging sequence. The uncaging sequence consists of a plurality of pulses with peak negative pressure ***P**_{U}* and a fixed number of cycles per pulse (*NOC*). These pulses are repeated with a pulse repetition frequency (*PRF*) for a total duration ***t**_{U}.* Subsequently a reperfusion period (FUS-OFF) with duration ***t**_{OFF}* permits reperfusion of UC-carriers. The entire sequence is then repeated for the duration of sonication.

### In vitro characterization of AU-FUS sequence

For *in vitro* characterization of the AU-FUS sequence, dye-loaded UC-carriers flowed through microdialysis tubing (13 kDa cut-off, single pass), embedded in low-melt agarose. The system was confocally aligned to an inverted water immersion 60x objective lens and a 2.5 MHz FUS transducer. The entire setup was inside a custom-made water tank, filled with deionized, degassed water.

Fluorescein-loaded UC-carriers flowed continuously through the dialysis tubing at speeds (10 µL/min, corresponding to 5 mm/s of flow speed) that mimicked the highest blood flow rates in brain capillaries. The carriers were then exposed to either commonly used BBB-opening FUS (standard-FUS sequence) or the novel AU-FUS sequences.

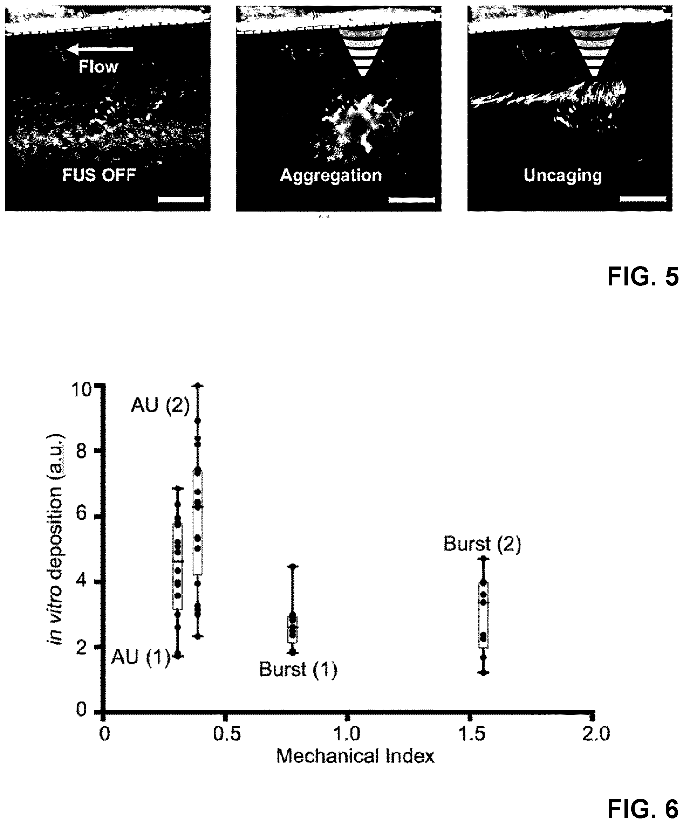

The effect of the novel AU-FUS sequences is illustrated in Fig. 5, which shows UC-carriers (white particles in the image) flowing through microdialysis tubing with no FUS (left). Aggregation (middle) and uncaging (right) sequences concentrate the UC-carriers and release the small molecules, respectively. Dashed line indicates wall of tubing; scale bar (white) = 50 µm.

Dye release into the agarose was measured both within the focus and outside the focus of the FUS transducer. Standard-FUS ultrasound sequences as well as multi-component sequences were used, with the parameters provided in Table 1 below.

**Table 1: FUS parameters P_{A}, P_{U} - peak negative pressure in megapascal (MPa) for aggregation and uncaging sequences, respectively (*accounting for -70% skull attenuation; see Methods), t_{A} - pulse duration (in milliseconds) of aggregation sequence, t_{U} - duration (in milliseconds) of uncaging sequence, t_{OFF}- duration (in milliseconds) of delay between the end of the uncaging sequence and the start of the following aggregation sequence, PRF - pulse repetition frequency in Hz, NOC - number of cycles.**

| **Condition** | ***P**_{A}* (MPa) | ***t**_{A}* (ms) | ***P**_{U}* (MPa) | ***t_{U}*** (ms) | ***t_{OFF}*** (ms) | ***PRF*** (Hz) | 25000 |
|---|---|---|---|---|---|---|---|
| Standard₁-FUS | 1.25 | - | - | - | - | 1 | 25000 |
| Standard₂-FUS | 2.5 | - | - | - | - | 1 | 25000 |
| Standard₃-FUS | 0.75* | - | - | - | - | 1 | 25000 |
| Standard₄-FUS | 1.5* | - | - | - | - | 1 | 25000 |
| AU₁-FUS *(in vitro)* | 0.3 | 500 | 0.5 | 90 | 300 | 30 | 10000 |
| AU₂-FUS *(in vitro)* | 0.38 | 500 | 0.63 | 90 | 300 | 50 | 10000 |
| AU₃-FUS *(in vitro)* | 0.25 | 1000 | 0.63 | 90 | 300 | 100 | 1000 |
| AU-FUS *(in vivo)* | 0.075 * | 1000 | 0.188 * | 90 | 300 | 100 | 1000 |

The performance of the AU-FUS sequence was compared to the performance of standard, single-component FUS sequences. Results are illustrated in Fig. 6, which shows the deposition efficiency of small molecules (measured as normalized fluorescence) using multi-component AU-FUS [AU1- FUS *(in vitro),* AU2-FUS *(in vitro)* and single-component standard-FUS [standard1-FUS, standard2-FUS] vs. peak negative pressure. See Table 1 for parameters. All data is represented as a box-and-whisker plot [min to max, showing all measured points].

The AU-FUS sequences not only delivered more molecules than the standard-FUS sequences, but also required several-fold lower ultrasound pressures than both the standard-FUS sequences and the reported fragmentation pressures of Ferrara and colleagues, thus having a much reduced damage potential for the vasculature or BBB when used *in vivo.* Even doubling the pressures of standard-FUS sequences did not result in greater deposition, suggesting the acoustic radiation forces generated by the first component of the novel AU-FUS sequences played a role in efficient delivery of the molecules.

### Optimization for in vivo experiments

When tested *in vivo* (with FUS-parameters adjusted to account for the attenuation of ultrasound waves by the skull), even the best *in vitro* optimized AU-FUS parameters [AU₁-FUS (*in vitro*) and AU₂-FUS (*in vitro*)] still caused weak BBB opening. Since neither *in vitro* deposition nor artificial BBB models sufficiently mimic *in vivo* BBB, capillaries, drug, and blood-plasma interactions, the AU-FUS pulse sequences were further optimized by iterations between *in vivo* and *in vitro* experiments. It is remarked that the *in vitro* studies were performed using commercially available microdialysis tubing, which is larger than the diameter of brain capillaries. Due to the large size of ultrasound transducers and the optical objective lenses, it is currently infeasible to image microparticles in brain capillaries during FUS. Many parameters were systematically varied, including the number of cycles, pulse-repetition frequencies, amplitudes for each component of AU-FUS, pulse-to-pulse delays (FUS OFF period), UC-carrier concentrations, and UC-carrier lipid chemistries and compositions.

Based on these optimizations, an *in vivo* AU-FUS sequence [AU-FUS (*in vivo*); see Table 1] was found that was able to deliver small molecules with extremely high efficiency while also completely avoiding BBB damage. The effects of the *in vivo* optimized AU-FUS sequence on UC-carriers using the *in vitro* setup were studied. It was observed that neither aggregation nor uncaging sequences alone were effective in releasing small molecules and that both components are essential for low-pressure drug delivery when ultrasound power levels as low as in the *in vivo* AU-FUS sequence are used.

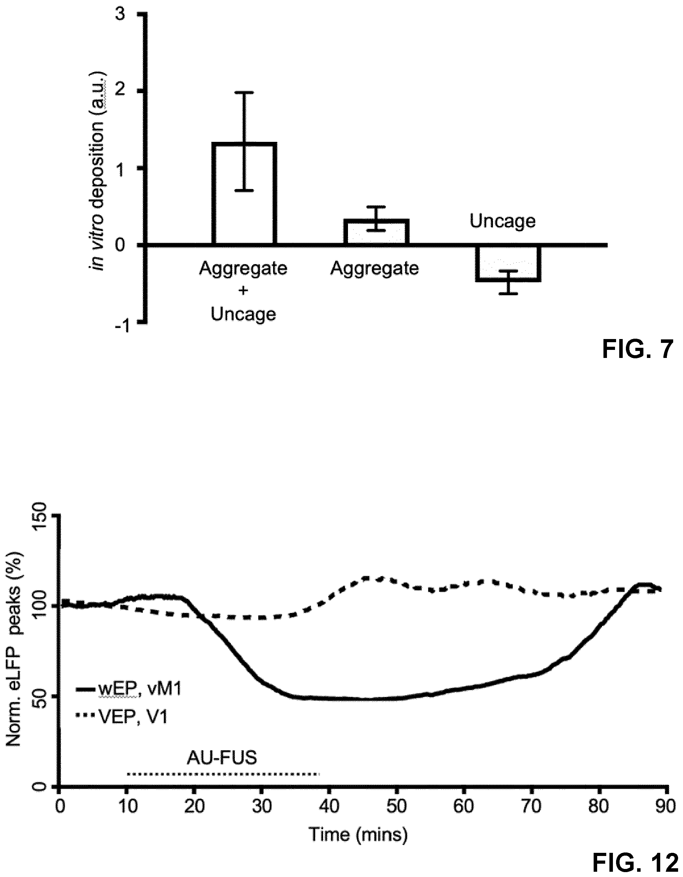

This is illustrated in Fig. 7, which shows the deposition efficiency of small molecule (measured as normalized fluorescence) using the final *in vivo*-optimized AU-FUS sequence components. Both the aggregation pulses and the fragmentation pulses are required for efficient release. All data is mean ± s.e.m. (n=16 for aggregate + uncage, n=11 for aggregate only, n=16 for uncage only).

### Receptor-specific focal modulation of a cortical network in vivo

The AU-FUS sequence [AU-FUS (*in vivo*); see Table 1] was tested *in vivo* by manipulating a specific cortical network without opening the BBB. Rat vibrissa motor cortex (vM1) receives whisker sensory information (-80%) through projections from vibrissa sensory cortex (vS1, "Barrel cortex"). When rodent whiskers are mechanically deflected, evoked activity propagates from brainstem via thalamus to vS1, and then to vM1. During simultaneous recordings the peaks of whisker-evoked potentials (wEPs) were observed first in vS1 and -3.0 ms later in vM1. It was tested whether inhibiting vS1 by the novel technique would suppress wEPs in vM1 in anesthetized rats. The FUS transducer was positioned above the intact skull and focused on vS1. Using a multielectrode array, the wEPs and the whisker-evoked multi-unit activity in vM1 were monitored online. In parallel, muscimol-loaded UC-carriers were continuously (i.v.) injected. The AU-FUS sequence was applied repeatedly on vS1 to aggregate the UC-carriers and to uncage muscimol. Muscimol is an agonist of ionotropic GABA_{A} receptors, the major receptor responsible for fast inhibitory transmission in the brain, which readily crosses the BBB.

### Setup for in vivo experiments

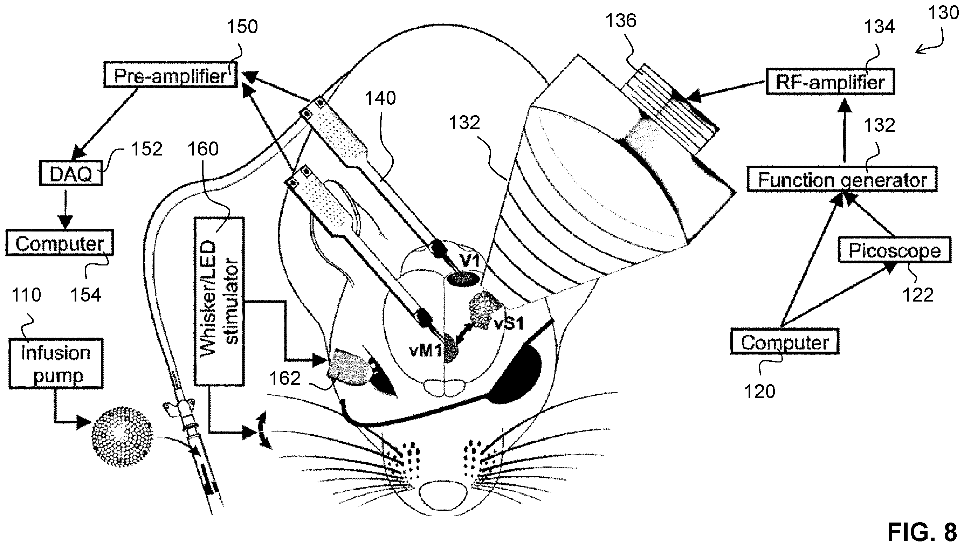

Figure 8 shows the experimental setup for *in vivo* drug delivery. The animal was anesthetized, a small craniotomy was performed over vM1 (TZ region) or V1 for inserting a recording probe 140, and a FUS transducer 136 was positioned on the intact skull above vS1 with the help of stereotaxic coordinates and coupled with sterile ultrasound gel and water collimator.

The FUS transducer 136 was operated by a function generator 132 via an RF amplifier 134. The function generator 132 was configured by a computer 120. The output from the function generator 132 was monitored by an oscilloscope 122.

Signals from the recording probes 140 were amplified by a pre-amplifier 150 and digitized using an analog-to-digital converter 152. Digitized signals were recorded by a computer 154, which may or may not be identical with computer 120.

Whiskers were mechanically stimulated by a whisker/LED stimulator 160. The whisker/LED stimulator 160 also drove an LED 162 for visually stimulating an eye of the animal. Contralateral whiskers or eye were mechanically deflected or visually stimulated, respectively, at 0.3 Hz.

Muscimol-loaded UC-carriers were injected intravenously through the tail vein using an infusion pump 110. vM1 and V1 recordings were performed on separate cohorts.

### Results of in vivo experiments

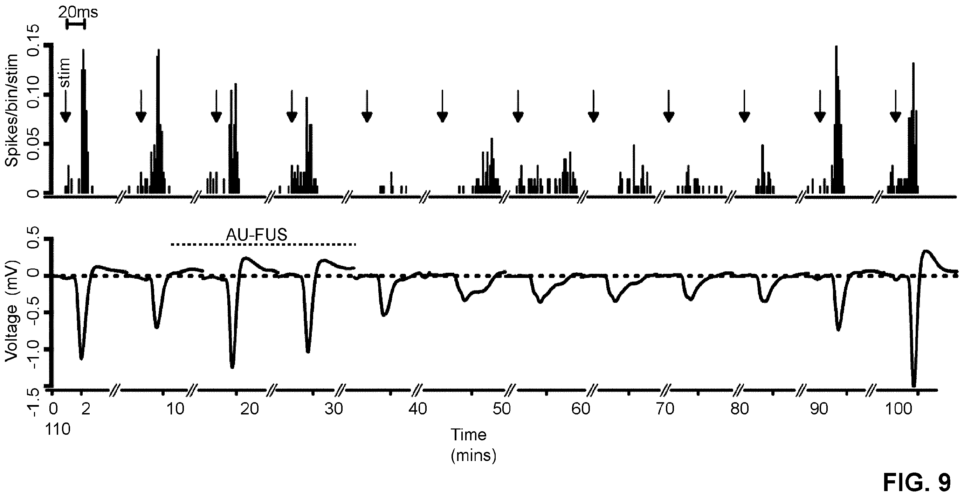

The experimental results showed that FUS-mediated delivery of muscimol inhibits vS1 and reduces the whisker evoked multi-unit activity and wEPs in its projection target vM1. This is illustrated in Fig. 9, which shows representative peri-stimulus time histograms (PSTHs, top, bin size 2 ms) and wEPs (bottom) upon focal aggregation of drug carriers (muscimol-loaded UC-carriers) and uncaging of muscimol from one experiment. Following 10 mins of baseline recording, the animal was injected with muscimol-loaded UC-carriers, 30 s later FUS was turned on. UC-carriers were injected over a period of -25 mins with an infusion pump at 0.2 mL/min. FUS was turned off 7 mins after the end of UC-carrier injection. wEPs were monitored online. Up to 62.62% inhibition (compared to baseline) was observed. PSTHs and wEPs recovered completely -75 mins after cessation of FUS. The wEPs and PSTHs were averaged over the 4 recording sites with the highest evoked responses over 2 mins windows. The stimulus onset is indicated by arrows.

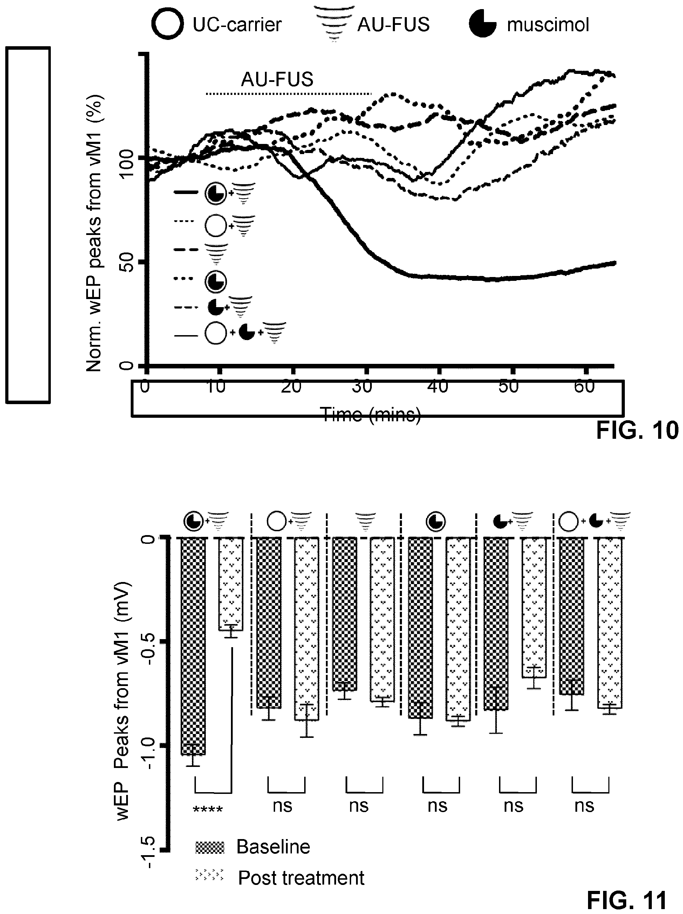

A complete set of control experiments was performed in order to confirm the specificity of the novel approach and to verify that only muscimol-loaded UC-carriers with AU-FUS (*in vivo*) results in local inhibition. The results of these control experiments are illustrated in Figs. 10 and 11. Fig 10 shows the time course of normalized wEPs (negative peak) in vM1. Results are shown for muscimol-loaded UC-carriers with AU-FUS (solid line, n = 6 rats x 4 recording sites), vehicle-loaded UC-carriers with AU-FUS (n = 6 rats x 4 recording sites), AU-FUS without UC-carrier injection (n = 9 experiments [from 5 rats] x 4 recording sites), muscimol-loaded UC-carrier injection without AU-FUS (n = 5 rats x 4 recording sites), systemic injection of free muscimol (250 ng) with AU-FUS (n = 5 rats x 4 recording sites), systemic injection of free muscimol (250 ng) and vehicle-loaded UC-carriers with AU-FUS (n = 5 rats x 4 recording sites). Evoked responses were averaged with a moving window (window size = 180 whisker deflections). All results are mean values. Fig. 11 shows wEP responses (negative peak), plotted as baseline vs. post-treatment. Baseline value is an average of 10 mins, and post-treatment value is an average of 30 mins (sampled randomly to match the number of data points collected for baseline) following the completion of AU-FUS or injection of muscimol/UC-carriers. Statistical comparison was performed pairwise for baseline vs treatment (Wilcoxon matched-pairs signed rank test): first column: muscimol-loaded UC-carriers + FUS, ****p < 0.0001; second column: vehicle-loaded UC-carriers + AU-FUS, p = 0.3902; third column: AU-FUS only, p = 0.2371; fourth column: muscimol-loaded UC-carriers only, p = 0.8695; fifth column: 250 ng free muscimol + AU-FUS, p = 0.1737; sixth column: vehicle-loaded UC-carriers + 250 ng free muscimol + AU-FUS, p = 0.2611. All data is mean ± s.e.m. No statistically significant changes in the wEPs in vM1 were observed under any of the five control conditions. Therefore, the reduction of wEPs in vM1 cannot be attributed to nonspecific effects of the FUS or the UC-carriers.

To rule out that the observed modulation of vM1 could be due to spreading of muscimol from vS1, signals were recorded from a cortical area close to vS1 but without significant connectivity with vS1: Muscimol was delivered to vS1 using AU-FUS, and the visually evoked potentials (VEPs) were measured from primary visual cortex (V1). No statistically significant changes in VEPs recorded from V1 were observed. This is illustrated in Fig. 12, which shows the time course of the normalized eLFP (negative peak) responses (vEP) in neighboring V1 (without significant connectivity with vS1), as compared to responses (wEP) in vM1. V1 and vM1 recordings were done on separate cohorts. The same ultrasound parameters were used for both vS1-vM1 and vS1-V1 paradigms.

The experimental results were also consistent with the initial estimates that small molecules cannot be spreading large distances to induce physiological responses: Spreading could happen through two different means. First, uncaged muscimol could perfuse through the capillaries to distant regions. Assuming the diffusion coefficient of a small molecule even in water as D = 1.5 x 10⁻⁵ cm² s⁻¹, and a capillary radius of r = 5 µm, small molecules would take τ = 16 ms (τ = r²/D) on average to reach to the capillary walls from anywhere within the capillary. Assuming an average blood flow speed in capillaries of v = 1.5 mm/sec, free muscimol is expected to flow only -25 µm (λ = τ^{∗}v) beyond its release site before it reaches the blood brain barrier. Even if free muscimol does not enter the brain tissue immediately and remains in the circulation, it cannot reach far away tissues before entry to veins because the maximal length of capillaries in the rat brain is only about 250 µm and drug uptake to the brain is mainly confined to the capillaries. Importantly, muscimol's concentration will be negligible after entering the vein and redistributing in systemic circulation. Second, muscimol could diffuse within the interstitial space. The diffusion coefficient of muscimol in rat barrel cortex is D = 8.7 x 10⁻⁶ cm² s⁻¹. Given that the maximum inhibition in vM1 occurs within 20 mins, muscimol can diffuse in tissue only up to ~1 mm. This diffusion distance has been confirmed by measuring the spread of fluorescent muscimol in the rat brain tissue. In deeper subcortical structures, the observed rostrocaudal spread of muscimol has been shown to be ∼1.7mm on timescales comparable to muscimol action in the present work. Since vM1 is ∼7 mm away from vS1 in rats, muscimol diffusion in the tissue from release site to recording site cannot be the cause of observed neuronal inhibition. Additionally, radial diffusion of muscimol also rapidly dilutes muscimol (∼d³) with distance (d) from the delivery locus, thus making its concentration too low to induce physiological responses. These estimates support the experimental finding above. Thus, drug delivery is highly local and the observed neuromodulations cannot be due to the spreading of uncaged drug.

### 1300-fold focal aggregation of drug by AU-FUS

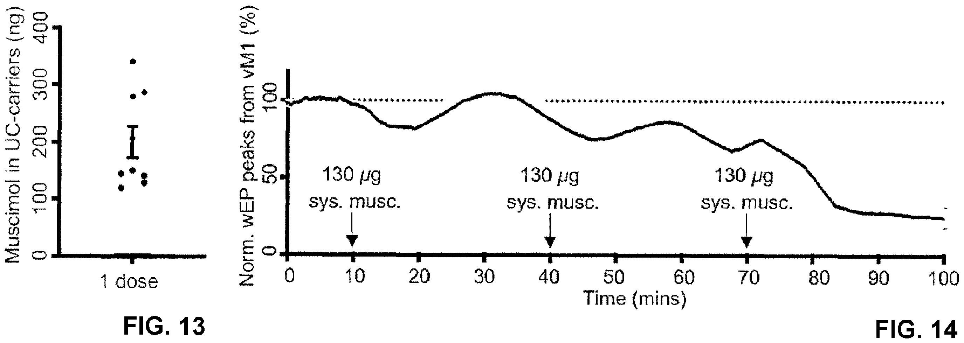

The total amount of muscimol injected in a single dose of AU-FUS treatment was -200 ng, as estimated using Liquid Chromatography-High Resolution Tandem Mass Spectroscopy (LC-HR-MS/MS). The results from LC-HR-MS/MS are shown in Fig. 13. All data is mean ± s.e.m, showing all points. Average = 199.7 ng, n = 9.

The effect of systemically administered muscimol on the wEPs in vM1 (without AU-FUS or UC-carriers) was tested. A comparable reduction of wEPs was observable only after systemically administering at least 1300 times the measured payload of the UC-carriers (at least 260 µg systemic muscimol). This is illustrated in Fig. 14, which shows the time course of wEPs (negative peak) in vM1 during systemic muscimol ("sys. muse.") injection (*i.v.*). Following 10 mins of baseline, 130 µg of muscimol (-650 times single muscimol-loaded UC-carrier injection dose) was manually injected over 1 min, every 30 mins (black arrows). Data is plotted as the moving average (window size = 180 whisker deflections). All data is mean ± s.e.m. n = 4 rats x 4 recording sites. Equivalent inhibition by AU-FUS would occur between 260 and 390 µg systemically.

This is consistent with the amount of systemic muscimol required for brain inactivation which is -1.6 mg/kg.

### Preservation of Blood-Brain Barrier Integrity and Normothermia

BBB integrity was assessed to determine the safety of the proposed sequence, as BBB-opening can be accompanied by inflammation and cell death. BBB opening was evaluated by measuring the extravasation of Evans Blue dye (EB) through IVIS spectrum epifluorescence imaging, Gadolinium (Gd)-enhanced T1-weighted Magnetic Resonance (MR) imaging, and extravasation of Immunoglobulin G (IgG) by immunohistochemical staining, none of which easily crosses the intact BBB. Tracer (EB, Gd, and IgG) extravasation was measured in regions of interest (ROIs) ipsilateral and contralateral to FUS in vS1 to determine BBB opening.

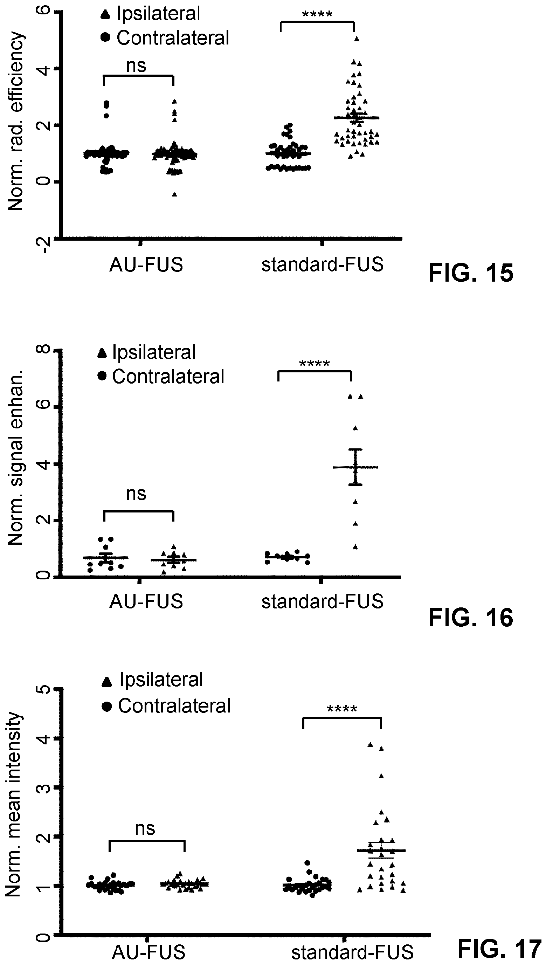

Results are illustrated in Figs. 15-17. Fig. 15 shows the normalized radiant efficiency of Evans blue extravasation within ROIs after AU-FUS (left) and standard-FUS (right); Fig. 16 shows the normalized signal enhancement by MRI-contrast agent extravasation within ROIs after AU-FUS and standard-FUS; and Fig. 17 shows a diagram illustrating normalized mean intensity values from ROI analysis of brightfield images of IgG stained brain sections after AU-FUS (left) and standard FUS (right).

There was no statistically significant difference in EB or IgG extravasation or Gd contrast enhancement when comparing ROIs contralateral and ipsilateral to FUS treatment site for the animals undergoing AU-FUS. In contrast, marked EB, Gd, and IgG labelling demonstrated profound BBB opening with standard-FUS parameters (standard₂-FUS in Table 1).

Since it is known that FUS can cause rapid temperature increases in the brain, which can have adverse effects on the BBB, the temperature within the AU-FUS focal volume was monitored. An average temperature increase of only 0.12 °C during the sonication was measured, which is within the normal range of temperature fluctuations in the awake behaving rats and is significantly below the threshold for alterations in BBB permeability, cell damage, or changes in cell activity.

### Passive cavitation detector responses show no spectral signatures of BBB opening

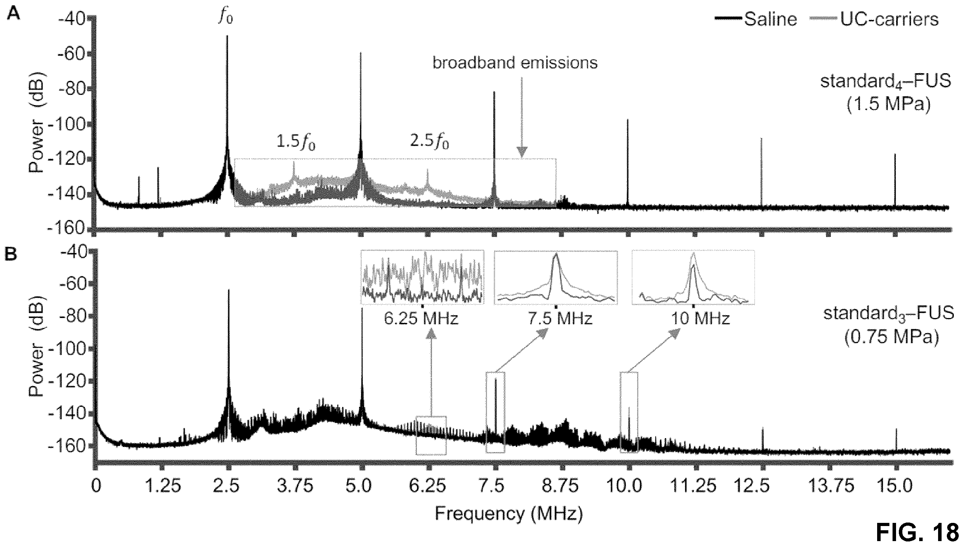

Passive cavitation responses of UC-carriers were measured under standard-FUS sequences at different pressures, and under the presently proposed AU-FUS sequence. The results are shown in Figs. 18-21.

Part A of Fig. 18 shows a Fast Fourier Transform (FFT) analysis of representative responses to the standard₄-FUS sequence. 60 s after starting IV injection of either saline or UC-carriers, the targeted brain region (AP = -4.67 mm, ML = -2.51 mm, DV = 2.3 mm) was sonicated with a 10 ms pulse at 1 Hz PRF for 5 mins. Signals were acquired using a passive cavitation detector. The first 2 ms of each pulse were used for FFT analysis after applying a Hamming window. Data is mean of 50 pulses. Broadband emissions (inside the box) and ultra-harmonics [i.e., non-integer harmonics at 3.75 MHz (1,5*f*₀) and 6.25 MHz (2.5*f*₀)] were detected only in the presence of UC-carriers (upper trace) as compared to saline (lower trace), indicating the presence of inertial cavitation.

Part B of Fig. 18 shows an FFT analysis of representative response to the standard₃-FUS sequence. 60 s after starting IV injection of either saline or UC-carriers, the targeted brain region (AP = -2.65 mm, ML = -2.03 mm, DV = 2.71 mm) was sonicated with a 10 ms pulse at 1 Hz PRF for 5 mins. The first 2 ms of each pulse were used for FFT analysis after applying a Hamming window. Data is mean of 50 pulses. Insets show ultra-harmonics and integer harmonics. Ultra-harmonics [3.75 and 6.25 MHz], but no broadband emissions are detected in the presence of UC-carriers (upper trace in the insets) as compared to saline (lower trace in the insets), indicating the presence of intense stable cavitation without inertial cavitation.

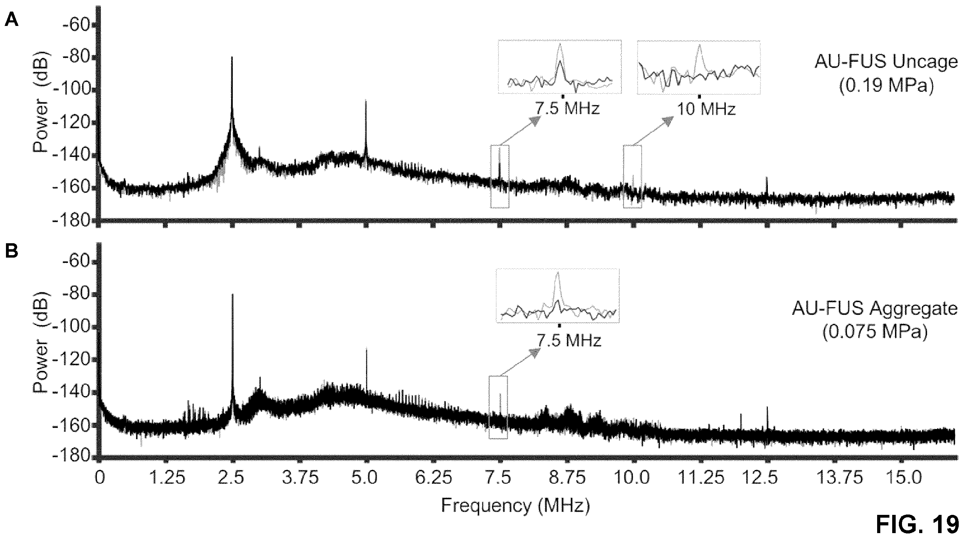

Part A of Fig. 19 shows an FFT analysis of a representative response to the AU-FUS Uncaging sequence, preceded by the AU-FUS Aggregation sequence. 60 s after starting IV injection of either saline or UC-carriers, the targeted brain region (AP = -2.65 mm, ML = +2.03 mm, DV = 2.71 mm) was sonicated with the AU-FUS sequence. The first pulse (1000 cycles) of the uncaging sequence impinging on the aggregated UC-carriers was used for data analysis after applying a Hamming window. Data is mean of 7 pulses. Insets show integer harmonics. Note that the FFT data has less samples due to 0.4 ms pulse width vs 2 ms in others. The results show neither ultra-harmonics nor broadband emissions in the presence of UC-carriers (upper trace) versus saline (lower trace), suggesting the absence of harmful inertial cavitation and strong reduction of stable cavitation.

Part B of Fig. 19 shows an FFT analysis of a representative response to the AU-FUS Aggregation sequence alone. 60 s after starting IV. injection of either saline or UC-carriers, the targeted brain region (AP = -2.65 mm, ML = +2.03 mm, DV = 2.71 mm) was sonicated with the AU-FUS sequence. The last 2 ms of the aggregation sequence were used for analysis after applying a Hamming window. Data is mean of 7 pulses. The inset shows an integer harmonic. Again, the results show neither ultra-harmonics nor broadband emissions in the presence of UC-carriers versus saline, suggesting no harmful inertial cavitation.

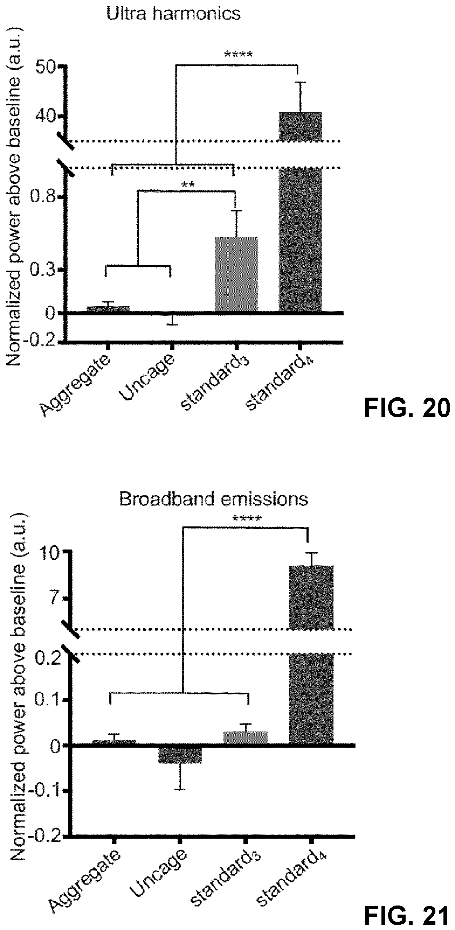

Figure 20 shows the calculated normalized power of ultra-harmonics at 1.5 and 2.5 *f*₀ above baseline (n = 3 rats x 50 pulses for standard_{3,4}-FUS, n = 3 rats x 7 pulses for AU-FUS sequence). All data is mean ± s.e.m. One-tailed, unpaired t-test with Welch's correction, Aggregate vs. standard₃-FUS, **p = 0.0052; Uncage vs. standard₃-FUS, **p = 0.0026; Aggregate vs. standard₄-FUS, ****p < 0.0001; Uncage vs. standard₄-FUS, ****p < 0.0001; standard₃-FUS vs. standard₄-FUS, ****p < 0.0001.

Figure 21 shows the calculated normalized power of broadband emissions above baseline (n = 3 rats x 50 pulses for standard_{3,4}-FUS, n = 3 rats x 7 pulses for AU-FUS sequence). All data is mean ± s.e.m. One-tailed, unpaired t-test with Welch's correction, Aggregate vs. standard₄-FUS, ****p < 0.0001; Uncage vs. standard₄-FUS, ****p < 0.0001; standard₃-FUS vs. standard₄-FUS, ****p < 0.0001. Pressures account for skull attenuation (see Table 1).

Upon injection of UC-carriers, the standard₄-FUS sequence (1.5 MPa) caused strong broadband emissions, indicating inertial cavitation, along with ultra-harmonic emissions (see part A of Fig. 18 and last column of Figs. 20 and 21). These results suggest that the standard₄-FUS sequence operates in the shock wave regime of BBB opening. This is a harmful BBB opening regime where microbubbles violently collapse, causing shock waves and microjet streams from microbubbles, leading to tissue damage and cell death along with BBB opening.

Sonicating with the standard₃-FUS sequence (0.75 MPa) caused ultra-harmonic emissions, which are signatures of stable cavitation, with negligible broadband emissions (see part B of Fig. 18 and in the penultimate column of Figs. 20 and 21). This suggests that the standard₃-FUS sequence operates in the "safe" regime of BBB opening. Evans Blue extravasation confirmed BBB opening.

In contrast, sonication of UC-carriers with the presently proposed AU-FUS sequence caused neither ultra-harmonics nor broadband emissions (see Fig. 19 and the first two columns of Figs. 20 and 21), indicating the absence of harmful inertial cavitation. As expected, only an increase in the amplitudes of integer harmonics was observed upon injection of UC-carriers. This is consistent with the observation that histological analysis does not indicate any BBB opening (cf. Figs. 15-17).

### Discussion

Technological advancements that enable safe and robust manipulation of specific neural circuits involved in disease pathologies can address both efficacy and molecular specificity challenges of existing treatments. In this study, a novel technique is demonstrated that allows efficacious and non-invasive modulation of specific brain circuits by spatially targeted delivery of receptor-specific small molecules. Several fundamental challenges were overcome to make this possible: 1) Devising a means to trap and concentrate sufficiently high numbers of UC- carriers in circulation, 2) Uncaging ample amounts of drug with minimal ultrasound energies to induce significant physiological responses, 3) Avoiding opening/damaging BBB and tissue heating, 4) Producing stable microbubble-liposome complexes suitable for use with multi- component FUS which can carry sufficient payloads with diverse physical properties, and 5) Identifying FUS sequences that do not cause nonspecific neuronal responses (i.e. without molecular specificity) due to the application of FUS alone (i.e. in the absence of UC-carriers).

The AU-FUS sequences concentrate the drug carriers not only along the radial axis of the capillaries (primary radiation forces), but also along the longitudinal axis of the capillaries (secondary radiation forces). While the applicant does not wish to be bound by theory, the aggregation component of the AU-FUS sequence likely utilizes secondary radiation forces, known as "Bjerknes forces", to drive the UC-carriers into close proximity to each other at low-pressure amplitudes. The dynamics of aggregated UC-carriers in a cluster are complex as they continue to volumetrically oscillate, coalesce, break up, and re-form repeatedly under continuous low-intensity ultrasound. While this physical response of UC-carriers could lead to enhanced drug delivery (as liposomes may be de-stabilized in this process), the exact mechanisms responsible for facilitating drug release from conjugated liposomes are difficult to elucidate due to the complex nature of UC-carrier aggregation, which is highly dependent on UC-carrier size distributions, concentrations, ultrasound frequencies and pressures. Additionally, the application of higher intensity fragmentation pulses in the second part of the AU-FUS sequence, as the microbubble shells destabilize, may promote better drug release from liposomes in UC-carrier clusters compared to individual UC-carriers, since gas from the microbubbles would leak out at high velocities, causing shear effects on the liposomal bilayer, thereby releasing its contents. It is also important to note that the present UC-carriers are expected to respond differently to FUS compared to microbubbles alone as liposome-attached bubbles have different physical properties than microbubbles alone.

The vS1-vM 1 circuit is a good model for *in vivo* validation of FUS-mediated delivery of drugs. First, the functional and anatomical connectivity between vS1-vM1 is well described. In this manner spatial specificity of FUS application and receptor specificity of drug loaded UC-carriers was demonstrated. Second, multiple studies demonstrate that inhibiting vS1 results in attenuation of evoked responses in vM1 as well. Finally, this circuit allowed to rule out the effects of uncaged muscimol spreading as a cause of the observed vM1 inhibition using a neighboring cortical area (V1) as control.

A major challenge in the chronic use of ultrasound-mediated drug release in the brain has been that previous approaches either required or caused unavoidable BBB opening. Recently, it has been shown that BBB opening can induce sterile inflammation under certain conditions, has strong effects on cell activity and behavior, and is implicated in neurodegenerative diseases. It is therefore highly desirable to achieve FUS-mediated drug release without compromising the BBB, especially for use in chronic treatments of many brain disorders. Therefore, passive cavitation monitoring of UC-carriers was performed to ensure the absence of inertial cavitation signals which otherwise indicate BBB opening or cell death. The intactness of BBB integrity was demonstrated by multiple measures: Evans Blue dye, Gadolinium extravasation through IVIS spectrum and MR imaging, respectively, as well as ImmunoglobulinG (IgG) immunohistochemical staining. To rule out even highly transient BBB opening, tracers (Evans Blue dye and Gadolinium) were also injected prior to sonication. No extravasation of the tracers was observed.

Recent studies by Airan and colleagues (see Background section) suggested that FUS-sensitive perfluoropentane (PFP) nanoemulsions (instead of the microbubbles+liposomes used here) might be used to deliver lipophilic-only agents to the brain without opening the BBB. However, unlike the gas-filled microbubbles used in the present study, nanoemulsions cannot be spatially concentrated due to their significantly lower responsiveness to acoustic radiation forces, making further improvements in their efficacy and specificity more challenging. Since the presently proposed UC-carriers require 1/1300 times or less drug than that required for systemic delivery to yield significant response, even if the payload is to be completely released systemically, it causes no detectable effect.

While the mechanistic understanding of psychiatric and neurological disorders has advanced in the preceding decades, translation of the knowledge or testing of hypotheses towards viable treatments has been almost non-existent. This has been in part due to the vast complexity and heterogeneity of the brain and an inability to selectively target regions and circuits of interest. Circuit-selective neuromodulation in humans may offer a strategic path towards effective treatment of many CNS disorders. Moreover, targeted delivery may offer a new avenue for small molecules that have failed due to toxicity or lack of efficacy, by allowing drugs to be delivered only to a desired region and possibly at higher concentrations than achievable through systemic delivery. Approved drugs should also benefit from focal delivery as this can likely eliminate, many if not all, side effects. The ability to enhance drugs' concentrations by 1300 fold over systemic levels demonstrates the power of our technology, and represents a fundamental leap forward in non-invasive targeted drug delivery.

Existing clinical ultrasound systems for targeting the brain are expensive and bulky, which would make chronic treatments challenging. However, they can be significantly scaled down with the use of integrated MEMS (Micro-Electro-Mechanical Systems) ultrasound transducers, which are capable of generating sufficient ultrasound power and can also be chronically implanted under the scalp or beneath/within the skull. Use of such technologies beneath the skull can also allow the use of higher frequency ultrasound waves (e.g. 2.5 MHz) with higher spatial resolution in clinical applications. The other option would be to use drugs with long lasting effects such as the NMDAR antagonist ketamine (recently approved as a rapid-onset antidepressant for patients with treatment resistant depression) which is efficacious for weeks following acute administration. Targeted delivery of ketamine could reduce its side-effects (such as psychotomimetic and perceptual disturbances, in addition to heart rate and blood pressure complications), while significantly enhancing its therapeutic index. While further refinement of our technology (e.g. reducing the sonication time required by further ultrasound sequences and/or drug loading and employing closed-loop, real-time passive cavitation detection for acoustic monitoring) can advance it to the clinic, the present approach presents a viable path forward, since individual chemical and ultrasound components of the presently proposed technology are already FDA approved. Regardless, the technology presented here enables the most efficacious, non-invasive, receptor-specific, millimeter-precision manipulation of brain circuits.

### Methods

### Ultrasound-Controlled drug carriers (UC-carriers)

Ultrasound controlled-drug carriers were created as follows. The UC-carriers contained a backbone of 1,2-distearoyl-sn-glycero-3-phosphocholine (DSPC) (Avanti Polar Lipids) and 1,2-distearoyl-sn-glycero-3-phosphoethanolamine (DSPE)-polyethylene glycol (PEG)2000 (Corden Pharma) in 90:5 molar ratio. The remaining 5% was DSPE-PEG5000-Thiol (SH) for liposomes and DSPE-PEG5000-Maleimide (MAL) (both from Nanocs) for the bubbles. The lipids were dissolved in chloroform and mixed in appropriate volumes to achieve a total concentration of 2 mg/mL for the bubbles and 10 mg/mL for liposomes. Chloroform was then evaporated under nitrogen and kept overnight under vacuum. The resultant lipid films were stored at -20°C till further use.

For the bubbles, the lipid films were rehydrated with 1x PBS containing 10% propylene glycol and 10% glycerol. The solution was then heated at 70°C for at least 30 mins, and bath sonicated for at least 20 mins or until the solution was clear. The headspace in the vial was filled with perfluorobutane (PFB; SynQuest Laboratories), and microbubbles were formed through a probe tip sonicator (70 % power; Branson SLPe with 3 mm tip). The microbubble solution was then size isolated by centrifugation at 300 x g for 3 mins, 3 times. After each centrifugation the wash solution was discarded and the remaining bubbles were resuspended in PBS:EDTA (1 mM EDTA; pH 6.5).

The liposome lipid films were rehydrated with 1x PBS, heated at 70°C for at least 30 mins, and bath sonicated for 3 hrs. Drug (muscimol from Hellobio, sodium fluorescein from Sigma-Aldrich) was added at a drug/lipid ratio of 0.3 and 15 freeze-thaw cycles were performed in liquid nitrogen and 37°C water bath, for 2.5 mins each (all steps performed in the dark for fluorescein). The liposomes yielded a mean size of -116 nm.

The resultant bubbles and liposomes were mixed and allowed to conjugate overnight at 4°C. Next day the solution was washed 2 times by centrifugation at 300 x g for 3 mins. The concentration and size distribution were analyzed in triplicate using Multisizer 3 (Beckman Coulter). The mean size of the UC-carriers was -1.7 µm.

### Animal preparation

Female Long Evans Rats (200-300 g, Charles Rivers Laboratories, Research Models and Service, Germany and Janvier Labs, Rodent Research Models and Associated Services, France) were used. The animals were housed in groups in standard IVC cages (Allentown), and had *ad libitum* access to food and water, and were on an inverted light cycle (12 hour dark/12 hrs light). All procedures were approved by the Veterinary Office, Canton Zürich, Switzerland.

### Surgery

Animals were anesthetized in an induction chamber with 4-5 % oxygenated isoflurane for 3-4 mins. They were then moved to a preparation area where the tail vein was catheterized with a winged 27G catheter (Terumo), and the head was shaved. 2 mg/kg Meloxicam (Metacam) and 7 mL/kg warmed Lactated Ringers solution (Fresenius Kabi, AG) were subcutaneously injected. The rat was moved to a stereotaxic frame (David Kopf), and an incision was performed on scalp to expose skull surface. A layer of eye cream was put on the eyes. A craniotomy was performed with a micro-drill above vM1 (Coordinates AP: 0-2.5 mm and ML: 0-2 mm, with respect to bregma), and dura was carefully opened. During the craniotomy, the skull was frequently flushed with Ringers solution (B. Braun) to prevent heating. After dura removal, a piece of gel foam (Pfizer) was put on brain and Ringers solution was regularly applied to keep the brain moisturized until electrode insertion.

### Whisker stimulation

Whiskers were cut to around 15 mm length. The 8-12 largest whiskers were inserted into a glass capillary tube which was attached to a piezo actuator (T223-H4CL-503X, Piezo Systems). The piezo actuator was shielded with a custom-made copper cover. The whiskers were deflected with 120 Hz cosine pulses (292 mm/s velocity, displaced 2.34 mm in 8 ms) which were generated in LabView (National Instruments) and converted into an analog signal (DAC NI, USB-6211) which then drove the piezo actuator. Stimulus presentation was synchronized with the electrophysiological recordings with a TTL signal at stimulus onset. Whiskers were continuously stimulated at a repetition rate of 0.3 Hz.

### FUS setup and transducer calibration

A custom-made transducer (Sonic Concepts) with 2.5 MHz center frequency, 40 mm diameter, 30 mm working distance/20.65 mm focal depth, and 0.5 x 0.5 x 2.5 mm theoretical focus was used, along with a custom impedance matching network for the transducer (Sonic Concepts). Calibration was done in a degassed and deionized water filled chamber with a 0.2 mm needle hydrophone (Acoustic Precision). The ultrasound pulses were generated with a function generator (Agilent 33210A, Keysight technologies) and PicoScope (3205B) oscilloscope and controlled by a custom MATLAB script. The signal was amplified 50 dB through a power amplifier (E&I 325LA).

### Electrophysiology and FUS drug delivery for vS1-vM1 measurements

All electrophysiological data were recorded with a RHD2000 system (Intan Technologies) with 30 kS/s sampling rate. All stereotaxic coordinates were determined with respect to bregma. A 32 channel Neuronexus probe (A2x16-10mm-100-500-177-A32, 15 or 50 µm thick) attached to a motorized 3D arm (StereoDrive-960HD, Neurostar), fixed outside of the stereotaxic frame, was inserted into the vM1 (Coordinates AP: 1-2 mm, ML: 0.5-1 mm, depending on the vasculature, DV: 1.5-2 mm from pia) at 50° to the coronal plane. The probe was initially inserted -250-500 µm below the cortical surface. The FUS transducer was integrated with an acoustic collimator which was filled with degassed and deionized water and contained a polystyrene film (McMaster-Carr) at the end. The collimator's shape was designed according to the FUS transducer's geometry so that it would not interfere with the FUS beam. This assembly was stereotaxically positioned such that the FUS focal volume was targeted to vS1 (Coordinates AP: -2.3 mm, ML: 6 mm, DV: 3.3 mm from skull surface) at a 30° angle with respect to the sagittal plane such that the focal volume of FUS beam targeted cortical layers of vS1. A sufficient amount of warmed (to body temperature) sterile ultrasound gel (Parker Laboratories) was put on the skull over vS1 for acoustic coupling. After positioning the FUS transducer, the recording probe was further inserted below cortical surface to reach a final DV position of 1.5-2 mm (tip). Following this, there was a period of about 1-2 hrs during which the wEP amplitudes stabilized. Baseline wEP responses were acquired for 10 mins, followed by the intravenous injection of small molecule- or vehicle- loaded UC-carriers (2 - 2.5 x 10⁹ total UC-carriers per animal) and/or muscimol (250 ng, Fig. 3C-3D) injection intravenously with an injector (Genie Touch Syringe Pump, Kent Scientific) at a speed of 0.2 mL/min. 30 seconds after the start of injection, FUS sonication was done for 25-30 mins (period of i.v. drug delivery). Electrophysiological data were recorded until at least 1 hour after the end of sonication to see the complete drug effect and recovery. Within half an hour of sonication, the animals were injected from i.v. with 1 mL of 0.5% Evans Blue (EB) dye to check for BBB integrity. EB dye was allowed to circulate for 30 mins to 2 hrs before transcardial perfusion and brain excision. Isoflurane was kept around 2.5 - 3% during all surgical procedures. During electrophysiological recordings and drug delivery it was maintained at around 1.5 - 2% to keep the anesthesia minimally low throughout the experiment. The anesthesia was regularly monitored visually with breathing rate and spontaneous wEP activity.

### Electrophysiology and FUS drug delivery for vS1-V1 measurements

The protocol used for vS1-V1 was the same as vS1-vM1 paradigm with the following exceptions: FUS sonication coordinates were changed to AP: -2.3 mm, ML: 6.5 mm, DV: 3.3 mm from skull surface and FUS angle was changed from 30° to 34° to create space for electrode insertion to V1. A more lateral region of vS1 was sonicated to achieve better coupling with skull with the new angling of the FUS transducer. Electrode insertion coordinates were changed for V1 recording to AP: -5.5-6.0 mm, ML: 3.2-3.5 mm, DV: 1.6-2.0 mm to ensure that recording is done from cortical layers of V1. The angle of the probe insertion was kept at 50° as in vM1 recording. Instead of wEPs, VEPs were recorded by visual stimulation. The amount of muscimol-loaded UC-carriers per animal was changed from 2 - 2.5 x 10⁹ to 2.5 - 3.0 x 10⁹ and sonication period was changed from 20-25 to 30-35 mins to show that even excessive muscimol delivery does not diffuse off-target brain circuits.

### Visual stimulation

A thin layer of eye cream was put on the contralateral eye. A 5 mm green LED was inserted in custom made black rubber cone. The cone was positioned on the contralateral eye such that the LED illuminates on the eye 3-4 mm away from the cornea. This configuration allowed sufficient intensity light stimulation to the eye, as the cone covered the eye and blocked any light from outside. The LED was triggered with a 10 ms TTL pulse which was generated in LabView (National Instruments) and buffered through a NI-USB-6211 (National Instruments) board. Stimulus presentation was synchronized with the electrophysiological recordings with the same TTL signal used for stimulation. The eye was continuously stimulated at a repetition rate of 0.3 Hz. The ipsilateral eye was covered with a black rubber after putting sufficient amount of eye cream. The whole recording session was done while all the lights in the room were turned off.

### Simultaneous vS1 and vM1 wEP recordings

Animals were prepared for craniotomy as indicated above and stereotaxic coordinates were determined with respect to bregma. An incision was made on the scalp and craniotomies were performed on vS1 (Coordinates AP: -2.3 mm, ML: 6 mm, DV: 1.1 mm from pia) then on vM1 (Coordinates AP: 1.5 mm, ML: 1 mm, DV: 0.8 mm from pia). vS1 was covered with gel foam, and continuously supplied with Ringers solution to keep the brain fresh until the vM1 craniotomy was completed. 8-12 whiskers were inserted into a capillary tube attached to a piezo stimulator. The first probe was inserted to vS1 with a 30° angle to the sagittal plane, 5 mins later the second electrode was inserted to vM1 parallel to the sagittal plane. Neuronexus, A4x8-5mm-100-200-177 probes were used for both recordings in the experiment. Once both probes were inserted, whiskers were continuously stimulated at 1 Hz, wEPs were allowed 1- 2 hrs to stabilize and responses were subsequently recorded for 8 mins.

### In vitro FUS characterization

UC-carriers (5 x 10⁸ MB/mL) flowed through a porous (13 kDa pore-size, 200 µm ID) microdialysis tube (132294, Spectra/Por) which was surrounded by agarose gel (0.6% in DI water + 0.9% NaCl, 16500500, UltraPure Invitrogen) in a custom-built channel. The custom-built channel that held the agarose gel had five marked sites, each of which was sequentially brought in the confocal alignment of a water immersion objective (CFI APO NIR 60X W, Nikon) and the FUS transducer in a water tank containing degassed and deionized water. The first four of the five sites in direction of flow were sonicated with the test FUS pulse sequences and the last site served as a control site. For either standard-FUS or AU-FUS characterizations, each site was sonicated for the same amount of time (5 mins). The tubing was then retracted from the agarose gel and each of the five agarose-gel sites were cut out and melted in heated (80°C) deionized water, and fluorescence was measured with a plate reader (the control site was subtracted from all readings) (Gen5 Microplate Reader, BioTek).

### 3D scanning of skull effects on FUS

Rat skulls were extracted, and tissue was removed from the surface before degassing in a chamber for 30 mins to remove any trapped air inside the skull cap. The skulls were then positioned inside a deionized and degassed water chamber and controlled manually by mounting them on a 3D stage (PT3/M, Thorlabs). The water chamber was at the base of a stereotaxic system (Neurostar). A metal pointer was mounted on the motorized arm of the stereotaxic system to find bregma. The FUS transducer subsequently replaced the metal pointer on the stereotaxic arm such that the focal point matches precisely with tip of metal pointer. The FUS transducer was then positioned such that the focus of the ultrasound was aligned at 4 mm rostral to bregma and 7 mm ventral from skull surface. A needle hydrophone (9 µm thick gold electrode PVDF film; 0.2 mm tip size, Precision Acoustics) was mounted on a motorized stage (PT3/M-Z8), and then moved to find focal point of the transducer. The hydrophone scanned an area (4 mm x 6 mm x 5 mm) in 3D with skull and (4 mm x 6 mm x 4 mm) without skull. The motors were moved with 100 µm step sizes to scan the AP and DV axes, and ML axis moved continuously to decrease time required for scanning, while data was collected with 14 µm resolution. ML values were then sampled every 100 µm. An optical displacement sensor (SICK, OD Mini) was used to find the position of ML motor, and an analog-to-digital-converter (ADC) (NI 6009) was used to gather sensor data to the computer. Hydrophone pressure readings were collected with a PicoScope (3205B), and the entire setup was controlled with a custom MATLAB script.

### Calculation of skull transmission factor

To measure the transmission factor of the skull, the peak negative pressure was measured with the hydrophone at the focal point of the transducer (P1), following which the skull was moved in between. Because the skull acts as a lens, the focal area changes slightly (depending on skull region and thickness). Hence, the hydrophone was repositioned to find the new focal area and peak negative pressure was measured again (P2). The transmission factor is calculated as P2/P1 x 100. For the brain region 4 mm rostral and 7 mm ventral to bregma, the transmission factor was 0.43 (57% loss), however for skull region above vS1 we expect -70% loss due to greater skull thickness. This data is in agreement with previous findings.

### Electrophysiology data analysis

All electrophysiological data analysis was done in Python, version 3.6, using custom scripts. For evoked potential (wEP→vM1 and VEP→V1) analysis, the raw data was low pass filtered (3rd order Butterworth filter) at 300 Hz. The wEPs and VEPs were extracted based on the time stamp of the whisker/visual stimulus. The waveforms were then corrected for amplitude offset by taking the mean of a 25 ms time-window preceding the stimulus onset. The peak negative value for the wEP and VEP was considered to be the amplitude of the wEP and VEP response and used for analysis and data visualization. The four recording sites (i.e. electrodes) with the highest response amplitudes were then automatically selected for each experiment. Extracellular spike detection and sorting was done with Klustakwik, an open source software. PSTHs were then extracted through a custom code in Python. For wEP and VEP analysis, moving averages were calculated for a window step size of 180 whisker deflections/ visual stimuli (moving step size is 1 deflection/visual stimulus). Responses were normalized for each electrode to the average response of the 10 min window preceding FUS. For statistical analysis, in order to keep the number of data points for baseline (10 mins, 152 peaks) and post treatment (30 mins, 457 peaks) same, 152 peak values from post treatment were randomly selected. Data was visualized using Prism 7.0 and 8.0.

### Measurement of brain temperature at the sonication site

A temperature probe (0.4 mm diameter, IT-21, Harvard Apparatus) was inserted through a 21G metal needle such that the tip of the probe stayed in the open cavity of the needle at the tip. This diameter (0.4 mm) was selected because it is smaller than the ultrasound wavelength (0.62 mm). A small craniotomy was performed on the skull in the following coordinates of AP: -2.3 mm, ML: 2-2.5 mm and the probe was inserted at a 52° angle. The probe was connected to a portable thermocouple thermometer with 0.1 °C resolution (Harvard Apparatus). The FUS traducer was positioned with a collimator and coupling gel on vS1 at 34°, and the temperature was allowed to stabilize for 1-2 hrs. The output of the thermocouple was digitized through an Analog-to-Digital Converter (ADC) (NI-6009, National Instruments). The corresponding output voltage from the ADC was converted to temperature and further analyzed using a custom MATLAB (Mathworks) script based on the probe's calibration sheet from the manufacturer. The data was collected at 10 Hz, a moving average was applied (window size of 10 s and step size of 0.1 s), and then further downsampled to 1 Hz for analysis. The data was visualized in Prism 8 (GraphPad).

### IVIS spectrum imaging

At the end of the experiment, animals were anesthetized with ketamine (100 mg/kg) and xylazine (10 mg/kg) prior to transcardial perfusion. Blood was cleared with phosphate-buffered saline (PBS) solution and animals were perfused with 4% paraformaldehyde solution (PFA; in PBS at pH 7). Brains were removed and placed in 4% PFA for at least 72-h before sectioning with a compresstome. Sections were cut at 100 µm thickness into a PBS bath and mounted onto microscope slides in MilliQ water. Sections were dried in the dark and subsequently imaged using the IVIS Spectrum (Living Image). The following parameters were used: Epi- Illumination, FOV: 6.6, FSTOP: 2, Binning: (M) 8, Exposure time: 1 s, Excitation: 465 nm, Emission: 680 nm, and scales are presented as radiant efficiency. Slides were imaged from ∼ bregma -2.28 ± 0.7 mm (AP) and ROI analysis was employed using dimensions slightly larger than the theoretical FUS focal volume (3.5 mm DV x 1.5 mm ML oval angled at 30°) at locations 6 mm (to top of ROI) from midline. Radiant efficiency values ((photons/sec/cm2/sr) / (µW/cm2)) for ROIs in vS1 for regions ipsilateral and contralateral to FUS were measured and values were normalized to the mean value of the contralateral side.

### MRI imaging

Contrast enhanced T1-weighted imaging was employed to visualize BBB disruption. Two groups of animals were compared (AU-FUS vs standard-FUS, n = 3 each). After sonication animals were transferred into the MR scanner (Bruker 7T PharmaScan). Pre-Contrast T1-weighted images were acquired (TE / TR: 4.5 ms / 146 ms; NEX = 3; FOV: 35 mm x 35 mm; matrix = 256 x 256; slice thickness: 0.5 mm; Flip Angle = 82°). These images were repeated (post-contrast) after injecting a bolus of the MRI contrast- agent Gd-DTPA (Omniscan) at 0.3 mL/kg. Additionally, TurboRARE anatomical images were acquired as a reference (TE / TR: 24 ms / 4095 ms; NEX = 10; echo spacing factor = 8; rare factor = 8; slice thickness = 0.45 mm; matrix: 180 x 120; FOV 20 mm x 12 mm). The animals were kept at a constant anesthesia level of 2.5% and sacrificed at the end of the experiment. A signal enhancement analysis was done. A region of interest (ROI) (1 mm x 1 mm) was drawn around 3 adjacent post-contrast T1-weighted slices, based on the FUS reference stereotactic coordinates, while excluding ventricles from the ROI. The difference in pre- and post-sonication T1-weighted images was calculated for ipsilateral and contralateral to FUS sites. The values for each group were pooled across animals, subsequently plotted and compared. In the case of Gd-DTPA injection before sonication, there was no pre-scan image, and the total signal intensity was measured for ROIs ipsilateral and contralateral to FUS, and values were normalized to the mean value for the contralateral side.

### IgG staining

Rats were treated with either AU-FUS or standard-FUS and remained under 2% isoflurane anesthesia for 3 hrs while maintaining body temperature with a thermometric heating blanket with rectal probe. Rats were injected with ketamine (100 mg/kg) and xylazine (10 mg/kg) prior to transcardial perfusion. Following perfusion, the brains were post-fixed for -18 hrs before preparing sections with a compresstome (50 µm thickness). Sections were blocked with 0.3% H₂O₂ in PBS for 10 mins at room temperature (RT), rinsed with PBS containing 0.25% triton X-100 (PBST), and blocked with PBST containing 10% normal goat serum. The tissue was then incubated with Biotinylated anti-rat IgG (Vector labs BA-9400) diluted 1:1500 in PBST overnight at 4 °C. Sections were rinsed 3 times with PBST, incubated in PBST containing horseradish peroxidase avidin D (diluted 1:8000; Vector labs A-2004) for 1 hr at RT, and washed 3 times with PBST before adding a solution containing 0.05% diaminobenzidine, 0.01% H₂O₂, and 0.3% imidazole in PBS for 10 mins at RT. The sections were immediately rinsed 3 times and mounted on glass slides before applying Fluoroshield with DAPI and sealing cover slips.

### IgG Image Acquisition and Analysis

All the slides were imaged using Nikon Eclipse TI microscope with Ander Neo sCMOS camera (DC-152Q-C00-FI) and NIS Elements software (v14.13.04 64 bit). Brightfield images were acquired using 4x objective (Plan Fluor 4x/0.13) and LED 0.8% intensity with 10 ms exposure and 1x1 binning. Single image tiles were acquired with 25% overlap, and automatic stitch blending, image registration, and shading correction was performed. All analysis was performed using FIJI. Three sets of 3 slices were analyzed, corresponding to the focal center (3 sections; ∼ -2.3 mm relative to bregma) and the anterior and posterior ends of the focal volume (3 sections each; 700 µM posterior or anterior to the focal center). Oval shaped ROIs (3.5mm x 1.5mm, 30° angle) 6 mm medial to midline at the surface of the cortex were added and average pixel intensity was measured. As increased staining decreases mean intensity, the normalized intensities are presented as the ROI contralateral to FUS treatment over the ROI ipsilateral to treatment.

### PCD Experiments and Analysis

Rats were anesthetized with isoflurane, the head was shaved, and 2 mg/kg meloxicam and 2 mL of Lactated ringers were injected subcutaneously. Animals were then fixed on a stereotaxic setup (Kopf) and a midline incision was made to expose the skull for brain coordinates. A metal pointer was attached to a motorized arm of stereotaxic system to find bregma. Sterilized ultrasound gel was then applied on the skull for coupling. A water box with ultrasound transparent polystyrene film at the bottom was positioned on the skull and filled with degassed water. The ultrasound transducer was then attached to the motorized stereotaxic arm with a custom-made metal pointer such that its focal point precisely targets bregma. The transducer was then moved to the target brain regions with the motorized arm using Neurostar software.

A single element FUS transducer (H-147, Sonic Concepts, USA) at 2.5 MHz frequency with focal volume of 0.51 x 0.51 x 3.28 mm was used. The transducer was calibrated with a 0.2 mm hydrophone (Acoustic Precision, UK) such that it generates pressures at the focal point identical to the custom-made transducer used for the drug delivery experiments. A broadband (10 kHz-15 MHz) passive cavitation detector (PCD, Y-107, Sonic Concepts, USA) was confocally aligned with the focal area of the transducer through a 20 mm center hole of the transducer. The data was amplified with a 20 dB RF amplifier (Ramsey Electronics), and digitized with the PicoScope (5242D, Pico Technology, UK) at 15 bits resolution with 125MS/s rate.

The data was recorded with the PicoScope's graphical user interface (PicoScope 6.14.10), and converted to MATLAB (.mat) format. For the standard_{3,4}-FUS sequences, the first 2 ms from the PCD was used in the analysis. For the AU-FUS sequence, the data was analyzed from two different segments separately since the AU-FUS sequence consists of two distinct sequences; AU-FUS Aggregation and AU-FUS Uncaging sequences. Because UC-carriers are expected to aggregate most strongly towards the end of the sequence, the last 2 ms of the AU-FUS Aggregation sequence was used for analysis. For the AU-FUS Uncaging sequence, since the first pulse sequence impinging on the aggregated bubbles is expected to give the strongest response, this pulse sequence (0.4 ms) is used for analysis. All the data was subjected to a Hamming window and plotted in the frequency domain after Fast Fourier Transform (FFT) in MATLAB 2015b.

50 kHz before and after the ultra-harmonics (3.75 and 6.25 MHz) was considered for area under-the-curve (AUC) calculations. For standard₄-FUS (1.5 MPa), the ultra-harmonics were detrended with a median filter to eliminate signal elevation due to the broadband emission background. For broadband emission calculations, 250 kHz before and after the integer and ultra-harmonics were excluded and the total range between 2.5 MHz to 10 MHz was covered.

### Extraction of muscimol from loaded UC-carriers for LC-HR-MS/MS quantification

Muscimol-loaded UC-carriers were prepared as stated above. After overnight conjugation of the microbubbles and liposomes, the solution was washed and centrifuged twice at 300g for 3 mins to remove any unencapsulated muscimol in PBS:EDTA (1 mM EDTA; pH 6.5). The final microbubble cake was resuspended in PBS:EDTA (1 mM EDTA; pH 6.5) and the concentration and size distribution were analyzed in triplicates using Multisizer 3 (Beckman Coulter). The total volume was also noted. 0.5 mL of absolute ethanol was added to the UC-carriers to dissolve the lipids and extract the muscimol. Following this, 2.5 mL running buffer (RB; 95% acetonitrile, 5% water, 0.1% formic acid) used in LC-HR-MS/MS detection was added. The solution was then bath sonicated at 70 °C. After this, 1.5 mL of the solution was centrifuged at 13,300 rpm for 10 mins, and the resultant supernatant was aliquoted in triplicate (400 µL) and frozen at -80 °C until LC-HR-MS/MS detection. The dilutions were noted and factored in while calculating the total amount of muscimol.

### Quantification of muscimol with LC-HR-MS/MS

The stock solutions of muscimol (HelloBio) and internal standard (methanamine hydrochloride, Sigma-Aldrich, ISTD) were prepared in a mixture of acetonitrile/H₂O 1:1 (v/v) (ULC-MS grade, Biosolve BV) at a concentration of 200 and 100 µg/mL, respectively. The stock solutions were stored at 4 °C until use. The solutions used for the quantification calibration curve were prepared as a dilution series at the concentrations of 1000, 500, 250, 50, 10, 2, and 0.5 ng/mL in acetonitrile/H₂O 1:1 (v/v) supplemented with ISTD at a final concentration of 200 ng/mL. The muscimol-loaded UC-carrier samples were diluted with acetonitrile/H₂O 1:1 (v/v) by a factor of 1:50. A volume of 5 µL was injected for quantification.

Liquid chromatography was performed on an UltiMate 3000 UHPLC (Thermo Fisher, Waltham, MA, USA) build from a binary RS pump, an XRS open autosampler, a temperature-controllable RS column department and a diode array detector, all from the series Dionex UltiMate 3000. Compound separation was achieved at 25°C on an ACQUITY UPLC Amide Column (100 Å, 1.7 µm, 2.1 x 100 mm; Waters, Milford, MA, USA). Eluent A consisted of H₂O and eluent B was acetonitrile, both acidified with 0.1% formic acid (VWR International bvba). The following conditions were applied for elution at a constant flow rate of 0.3 mL: (i) linear decrease starting from 90% to 50% B during 3.5 min; (ii) switch to 10% B from 3.5 to 3.7 min (iii) holding 10% B until 7.0 min (iv) change until 7.2 min to the starting conditions of 90% B; (v) equilibration for 2.8 min until the next measurement run.

Mass spectrometry was conducted on a QExactive quadrupole-Orbitrap mass spectrometer (Thermo Fisher Scientific, Waltham, MA, USA) equipped with a heated ESI source operating under following conditions: needle voltage of 3.5 kV, sheath, auxiliary and sweep gas (N₂) flow rates of 30, 15 and 0 (arbitrary units), respectively. The capillary and the auxiliary gas heater temperature amounted 280 °C and 250 °C, respectively. The data independent MS/MS mode (DIA) in positive ionization mode was selected including an inclusion list of the precursor ion corresponding to protonated molecules of muscimol (m/z 115.05020, 62 CE [collision energy], tR = 2.25-5.00 min) and ISTD (m/z 113.07094, 20 CE, tR = 0-2.25 min). A precursor ion isolation window of 3.0 m/z and a resolution of 70'000 at full width at half maximum (FWMH) were selected together with a maximum IT of 400 ms and an AGC target of 2 x 10⁵. The ion chromatograms corresponding to the signals of the fragment ions of muscimol and the ISTD were extracted at m/z 98.02-98.03 and 96.04-96.05, respectively. Xcalibur 4.1 and QuanBrowser 4.1 (Thermo Fisher Scientific) software were employed for data acquisition, and for peak-area integration and quantitation, respectively.

The recovery was estimated by first adding the standard solution containing 4.86 ng/mL muscimol in a 50x diluted sample containing 1.527 ng/mL. A spiked concentration of 6.32 ng/mL was obtained (98.6% recovery). In the second standard addition, 19.44 ng/mL were added to another sample containing 1.589 ng/mL. A spiked concentration of 22.212 ng/mL was obtained in this case (106.1% recovery).

Quantification was performed with the addition of the internal standard and the calibration curves were constructed by least-squares linear regression analysis. Thereby, peak area ratios of the signals from the analyte and the internal standard were plotted against the concentration of the analyte. The set of calibrators at 0.5, 2, 10, 50, 250, 500, and 1000 ng/mL concentration were measured to determine the dynamic range and the linearity of the quantification method. The quadratic fitting and the weighting function of 1/X2 were selected and correlation coefficient values R2 > 0.999 was obtained. A deviation below 5% was obtained by comparing the weighted and the measured concentrations.

### Statistical Analysis

Non-parametric statistical tests (pairwise Mann-Whitney rank sum test, or Wilcoxon matched-pairs signed rank test) were performed for electrophysiological data and imaging data. All statistical analysis was performed using GraphPad Prism version 7 and 8 for Mac, GraphPad Software, San Diego, California USA.

### Modifications

While the invention has been described in conjunction with specific embodiments, it is to be understood that the invention is not restricted to these embodiments, and that many modifications are possible without leaving the scope of the present invention. In particular, it is possible to apply the uncaging FUS sequence without prior application of the aggregation FUS sequence. It is also possible to deliberately open the BBB before application of the AU-FUS sequence in order to enhance delivery of drugs that are not able to cross the intact BBB. Other drug carriers than those described may be used, in particular, drug carriers comprising other types of microbubbles with or without liposomes attached to them.

## Claims

1. A system for enhancing localized delivery of a diagnostic or therapeutic agent to a human or animal body using focused ultrasound (FUS), the system comprising:
a source of ultrasound-controllable drug carriers (10), each drug carrier comprising an ultrasound-sensitive microbubble (20), the microbubble (20) comprising a microbubble shell and a gas encapsulated by the microbubble shell, the microbubble (20) being loaded with a diagnostic or therapeutic agent, preferably each drug carrier comprising a plurality of microparticles or nanoparticles attached to the microbubble, the microparticles or nanoparticles comprising the diagnostic or therapeutic agent;
an administration device (110) for administering the ultrasound-controllable drug carriers (10) into a blood vessel of the human or animal body;
a focused ultrasound (FUS) source (130) for creating FUS radiation in a target region of the human or animal body; and
a controller (120) configured to operate the focused ultrasound source (130) to apply an aggregation FUS sequence (40) to the drug carriers in the target region, the aggregation FUS sequence (40) generating radiation forces that cause the drug carriers (10) to aggregate inside the blood vessel, preferably both along a radial direction and a longitudinal direction of the blood vessel.

2. The system of claim 1, wherein the controller (120) is configured to operate the focused ultrasound source (130) to apply the aggregation FUS sequence at FUS power levels below a threshold power level such that broadband emissions and preferably non-integer harmonics are essentially absent from an emission spectrum of the drug carriers (10) in the target region.

3. The system of any one of the preceding claims, wherein the controller (120) is further configured to operate the focused ultrasound source (130) to apply an uncaging FUS sequence (50) to the target region, the uncaging FUS sequence (50) being applied subsequent to the aggregation FUS sequence (40), the uncaging FUS sequence (50) causing the diagnostic or therapeutic agent to be released from drug carriers (10) in the target region.

4. The system of claim 3, wherein the controller (120) is configured to operate the focused ultrasound source (130) to apply both the aggregation FUS sequence (40) and the uncaging FUS sequence (50) at FUS power levels below a threshold power level such that such that broadband emissions and preferably non-integer harmonics are essentially absent from an emission spectrum of the drug carriers (10) in the target region.

5. A system for enhancing localized delivery of a diagnostic or therapeutic agent to a human or animal body using focused ultrasound (FUS), the system comprising:
a source of ultrasound-controllable drug carriers (10), each drug carrier comprising an ultrasound-sensitive microbubble (20), the microbubble (20) comprising a microbubble shell and a gas encapsulated by the microbubble shell, the microbubble (20) being loaded with a diagnostic or therapeutic agent, preferably each drug carrier comprising a plurality of microparticles or nanoparticles attached to the microbubble, the microparticles or nanoparticles comprising the diagnostic or therapeutic agent;
an administration device (110) for administering the ultrasound-controllable drug carriers (10) into a blood vessel of the human or animal body;
a focused ultrasound (FUS) source (130) for creating FUS radiation in a target region of the human or animal body; and
a controller (120) configured to operate the focused ultrasound source (130) to apply an uncaging FUS sequence (50) to the drug carriers in the target region, the uncaging FUS sequence (40) causing the diagnostic or therapeutic agent to be released from the drug carriers (10),
wherein the controller (120) is configured to operate the focused ultrasound source (130) to apply the uncaging FUS sequence (50) at FUS power levels below a threshold power level such that such that broadband emissions and preferably non-integer harmonics are essentially absent from an emission spectrum of the drug carriers in the target region.

6. The system of claim 4 or 5,
wherein the administration device (110) is configured for administering the ultrasound-controllable drug carriers (10) into a blood vessel of the brain of the human or animal body,
wherein the target region is a region of said brain,
wherein the controller (120) is configured to operate the focused ultrasound source (130) to apply the uncaging FUS sequence (50) at FUS power levels below a threshold power level such that the blood-brain barrier remains intact, and
wherein preferably the diagnostic or therapeutic agent is capable of crossing the blood-brain barrier without opening of the blood-brain barrier, in particular, wherein the diagnostic or therapeutic agent preferably is a small molecule having a molecular mass of not more than 900 daltons, or a virus or antibody that is capable of crossing the blood-brain barrier by an active mechanism.

7. A method for enhancing localized delivery of a diagnostic or therapeutic agent to a human or animal body using focused ultrasound (FUS), the method comprising:
a) administering ultrasound-controllable drug carriers (10) into a blood vessel of the human or animal body, each drug carrier (10) comprising an ultrasound-sensitive microbubble (20), the microbubble (20) comprising a microbubble shell and a gas encapsulated by the microbubble shell, the microbubble (20) being loaded with a diagnostic or therapeutic agent, preferably each drug carrier comprising a plurality of microparticles or nanoparticles attached to the microbubble, the microparticles or nanoparticles comprising the diagnostic or therapeutic agent; and
b) aggregating the drug carriers (10) in a target region inside the blood vessel by exposing the drug carriers (10) to radiation forces, the radiation forces being generated by applying an aggregation FUS sequence (40) to the target region, the drug carriers (10) preferably being aggregated in the target region both along a radial direction and a longitudinal direction of the blood vessel.

8. The method of claim 7, wherein the aggregation FUS sequence (40) is applied at FUS power levels below a threshold power level such that broadband emissions and preferably non-integer harmonics are essentially absent from an emission spectrum of the drug carriers (10) in the target region.

9. The method of claim 7 or 8, further comprising:
c) releasing the diagnostic or therapeutic agent from the drug carriers (10) after aggregation by application of an uncaging FUS sequence (50) to the target region.

10. The method of claim 9, wherein both the aggregation FUS sequence (40) and the uncaging FUS sequence (50) are applied at FUS power levels below a threshold power level such that broadband emissions and preferably non-integer harmonics are essentially absent from an emission spectrum of the drug carriers (10) in the target region.

11. A method of enhancing localized delivery of a diagnostic or therapeutic agent to a human or animal body using focused ultrasound (FUS), the method comprising:
a) administering ultrasound-controllable drug carriers (10) into a blood vessel of the human or animal body, each drug carrier (10) comprising an ultrasound-sensitive microbubble (20), the microbubble (20) comprising a microbubble shell and a gas encapsulated by the microbubble shell, the microbubble (20) being loaded with a diagnostic or therapeutic agent, preferably each drug carrier comprising a plurality of microparticles or nanoparticles attached to the microbubble, the microparticles or nanoparticles comprising the diagnostic or therapeutic agent; and
b) releasing the diagnostic or therapeutic agent from the drug carriers (10) by application of an uncaging FUS sequence (50) to a target region, wherein the uncaging FUS sequence (50) is applied at FUS power levels below a threshold power level such that broadband emissions and preferably non-integer harmonics are essentially absent from an emission spectrum of the drug carriers (10) in the target region.

12. The method of claim 10 or 11,
wherein the ultrasound-controllable drug carriers (10) are administered into a blood vessel of the brain of the human or animal body,
wherein the target region is a region of said brain,
wherein the uncaging FUS sequence (50) is applied at FUS power levels below a threshold power level such that the blood-brain barrier remains intact, and
wherein preferably the diagnostic or therapeutic agent is capable of crossing the blood-brain barrier without opening of the blood-brain barrier, in particular, wherein the diagnostic or therapeutic agent preferably is a small molecule having a molecular mass of not more than 900 daltons, or a virus or antibody that is capable of crossing the blood-brain barrier by an active mechanism.

13. The method of any one of claims 7 to 12, comprising:
recording ultrasound emissions from the drug carriers (10) in the target region;
obtaining a frequency spectrum of the recorded ultrasound emissions; and
setting FUS power levels such that broadband emissions and preferably non-integer harmonics are essentially absent from the obtained frequency spectrum.

14. Use of an ultrasound-controllable drug carrier (10) for enhancing localized delivery of a diagnostic or therapeutic agent to a human or animal body, the ultrasound-controllable drug carrier (10) comprising an ultrasound-sensitive microbubble (20), the microbubble (20) comprising a microbubble shell and a gas encapsulated by the microbubble shell, the microbubble (20) being loaded with a diagnostic or therapeutic agent, preferably each drug carrier comprising a plurality of microparticles or nanoparticles attached to the microbubble, the microparticles or nanoparticles comprising the diagnostic or therapeutic agent, the enhancement of localized delivery of the diagnostic or therapeutic agent being carried out by a method according to any one of claims 7 to 13.

15. An ultrasound-controllable drug carrier (10) for use in a method for enhancing localized delivery of a diagnostic or therapeutic agent to a human or animal body according to any one of claims 7 to 13, the ultrasound-controllable drug carrier (10) comprising an ultrasound-sensitive microbubble (20), the microbubble (20) comprising a microbubble shell and a gas encapsulated by the microbubble shell, the microbubble (20) being loaded with a diagnostic or therapeutic agent, preferably each drug carrier comprising a plurality of microparticles or nanoparticles attached to the microbubble, the microparticles or nanoparticles comprising the diagnostic or therapeutic agent.
